# EUROPÄISCHE PATENTSCHRIFT

(11) **EP 2 606 025 B1**
(45) Veröffentlichungstag und Bekanntmachung des Hinweises auf die Patenterteilung: **12.10.2016**
(21) Anmeldenummer: 11752156.7
(22) Anmeldetag: 19.08.2011
(51) Int. Cl.: C07C 51/15, C07C 57/20, B01J 31/18, C07D 471/02

(54) **VERFAHREN ZUR HERSTELLUNG EINER PROPIOLSÄURE ODER EINES DERIVATES DAVON**
PROCESS FOR PREPARING A PROPIOLIC ACID OR A DERIVATIVE THEREOF
PROCÉDÉ DE PRÉPARATION D'UN ACIDE PROPIOLIQUE OU D'UN DÉRIVÉ DE CELUI-CI

(30) Priorität: 20.08.2010 DE 102010034922
(43) Veröffentlichungstag der Anmeldung: 26.06.2013
(73) Patentinhaber: BASF SE, 67056 Ludwigshafen am Rhein (DE)
(72) Erfinder: GOOSSEN, Lukas, J., 67663 Kaiserslautern (DE); RODRIGUEZ GARRIDO, Nuria, E-28409 Madrid (ES); MANJOLINHO, Filipe, 67122 Altrip (DE); LANGE, Paul, P., San Diego CA 92122 (US)
(86) Internationale Anmeldenummer: PCT/EP2011/064281
(87) Internationale Veröffentlichungsnummer: WO 2012/022801

(56) Entgegenhaltungen:
- WO-A1-2011/075087
- DE-C1- 19 809 532
- D. YU ET AL: "Copper- and copper-N-heterocyclic carbene-catalyzed CboxhH activating carboxylation of terminal alkynes with CO2 at ambient conditions", PROCEEDINGS OF THE NATIONAL ACADEMY OF SCIENCES, Bd. 107, Nr. 47, 23. November 2010 (2010-11-23), Seiten 20184-20189, XP55014733, ISSN: 0027-8424, DOI: 10.1073/pnas.1010962107
- LUKAS J. GOOSSEN ET AL: "Synthesis of Propiolic Acids via Copper-Catalyzed Insertion of Carbon Dioxide into the CH Bond of Terminal Alkynes", ADVANCED SYNTHESIS & CATALYSIS, Bd. 352, Nr. 17, 22. November 2010 (2010-11-22), Seiten 2913-2917, XP55014738, ISSN: 1615-4150, DOI: 10.1002/adsc.201000564
- PETER WIPF ET AL: "Synthesis of the C 1' -C 11' Segment of Leucascandrolide A", THE JOURNAL OF ORGANIC CHEMISTRY, Bd. 66, Nr. 9, 1. Mai 2001 (2001-05-01), Seiten 3242-3245, XP55014759, ISSN: 0022-3263, DOI: 10.1021/jo005787q
- IAN D. POTTER ET AL: "Phosphine displacement reactions by some alkyl-substituted 1,10-phenanthrolines with bis(triphenylphosphine)copper(I)tetrahydro borate", INORGANICA CHIMICA ACTA, Bd. 207, Nr. 2, 1. Mai 1993 (1993-05-01), Seiten 165-173, XP55014771, ISSN: 0020-1693, DOI: 10.1016/S0020-1693(00)90706-0
- DATABASE CA [Online] CHEMICAL ABSTRACTS SERVICE, COLUMBUS, OHIO, US; CROSBY, G. A. ET AL: "Electronic excited states of copper(I) substituted-1,10-phenanthroline and substituted-phosphine mixed-ligand complexes", XP002665815, gefunden im STN Database accession no. 1989:504853 & CROSBY, G. A. ET AL: "Electronic excited states of copper(I) substituted-1,10-phenanthroline and substituted-phosphine mixed-ligand complexes", PROCEEDINGS OF SPIE-THE INTERNATIONAL SOCIETY FOR OPTICAL ENGINEERING , 1054(FLUORESC. DETECT. 3), 214-16 CODEN: PSISDG; ISSN: 0277-786X, 1989,
- DATABASE CA [Online] CHEMICAL ABSTRACTS SERVICE, COLUMBUS, OHIO, US; YERSIN, HARTMUT ET AL: "Luminescent cyclometalated and chelate metal complexes containing bulky nido-carborane ancillary ligands as components for organic light-emitting devices", XP002665816, gefunden im STN Database accession no. 2009:20573 & WO 2009/003700 A1 (MERCK PATENT G.M.B.H., GERMANY; UNIVERSITAET REGENSBURG) 8. Januar 2009 (2009-01-08)
- DATABASE CA [Online] CHEMICAL ABSTRACTS SERVICE, COLUMBUS, OHIO, US; CASADONTE, DOMINICK J., JR. ET AL: "Hindered internal conversion in rigid media. Thermally nonequilibrated 3IL and 3CT emissions from [Cu(5-X-phen)(PPh3)2]+ and [Cu(4,7-X2-phen)(PPh3)2]+ systems in a glass at 77 K", XP002665817, gefunden im STN Database accession no. 1987:40740 & CASADONTE, DOMINICK J., JR. ET AL: "Hindered internal conversion in rigid media. Thermally nonequilibrated 3IL and 3CT emissions from [Cu(5-X-phen)(PPh3)2]+ and [Cu(4,7-X2-phen)(PPh3)2]+ systems in a glass at 77 K", JOURNAL OF THE AMERICAN CHEMICAL SOCIETY , 109(2), 331-7 CODEN: JACSAT; ISSN: 0002-7863, 1987,
- DATABASE CA [Online] CHEMICAL ABSTRACTS SERVICE, COLUMBUS, OHIO, US; YANG, LI ET AL: "Structures, electronic states and electroluminescent properties of a series of CuI complexes", XP002665818, gefunden im STN Database accession no. 2005:494826 & YANG, LI ET AL: "Structures, electronic states and electroluminescent properties of a series of CuI complexes", EUROPEAN JOURNAL OF INORGANIC CHEMISTRY , (10), 1867-1879 CODEN: EJICFO; ISSN: 1434-1948, 2005,
- DATABASE CA [Online] CHEMICAL ABSTRACTS SERVICE, COLUMBUS, OHIO, US; SAKAGAMI, MEGUMU ET AL: "Pigment sensitized photoelectrochemical cells", XP002665819, gefunden im STN Database accession no. 2000:223906 & JP 2000 100482 A (MATSUSHITA ELECTRIC INDUSTRIAL CO., LTD., JAPAN) 7. April 2000 (2000-04-07)
- DATABASE CA [Online] CHEMICAL ABSTRACTS SERVICE, COLUMBUS, OHIO, US; KONG, ZHIGUO ET AL: "Highly sensitive organic ultraviolet optical sensor based on phosphorescent Cu(I) complex", XP002665820, gefunden im STN Database accession no. 2006:1168976 & KONG, ZHIGUO ET AL: "Highly sensitive organic ultraviolet optical sensor based on phosphorescent Cu(I) complex", APPLIED PHYSICS LETTERS , 89(16), 161112/1-161112/3 CODEN: APPLAB; ISSN: 0003-6951, 2006,
- DATABASE CA [Online] CHEMICAL ABSTRACTS SERVICE, COLUMBUS, OHIO, US; TSUKUDA, TOSHIAKI ET AL: "Luminescence of copper(I) dinuclear complexes bridged by diphosphine ligands", XP002665821, gefunden im STN Database accession no. 2006:256724 & TSUKUDA, TOSHIAKI ET AL: "Luminescence of copper(I) dinuclear complexes bridged by diphosphine ligands", BULLETIN OF THE CHEMICAL SOCIETY OF JAPAN , 79(2), 288-290 CODEN: BCSJA8; ISSN: 0009-2673, 2006,
- SHIGEYOSHI SAKAKI ET AL: "Successful photocatalytic reduction of methylviologen (MV2+) with [Cu(NN)(PPh3)2]+ (NN = 2,9-dimethyl-1,10-phenanthroline or 4,4',6,6'-tetramethyl-2,2'-bipyridine) upon near-UV-light irradiation and a novel solvent effect on its catalytic activity", INORGANIC CHEMISTRY, Bd. 25, Nr. 14, 1. Juli 1986 (1986-07-01), Seiten 2330-2333, XP55014898, ISSN: 0020-1669, DOI: 10.1021/ic00234a010

## Beschreibung

Die Erfindung betrifft ein Verfahren zur Herstellung von Propiolsäuren durch Umsetzung von terminalen Alkinen mit Kohlendioxid in Gegenwart eines Übergangsmetallkatalysators.

Propiolsäuren sind vielseitige Synthesebausteine z.B. in Cycloadditionen oder Hydroarylierungsreaktionen. Propiolsäuren erlauben die Synthese von vielerlei heterocyclischen Verbindungen wie Cumarine, Flavone und Indole (siehe hierzu beispielsweise a) B. M. Trost, F. D. Toste, K. Greenman, J. Am. Chem. Soc. 2003, 125, 4518-4526; b) T. Kitamura, Eur. J. Org. Chem. 2009, 1111-1125; c) M. Bararjanian, S. Balalaie, F. Rominger, B. Movassagh, H. R. Bijanzadeh, J. Org. Chem. 2010, 75, 2806-2812.). Darüberhinaus werden sie in decarboxylierenden Kreuzkupplungen zur Synthese von Alkinylarenen oder Aminoalkinen eingesetzt (a) J. Moon, M. Jeong, H. Nam, J. Ju, J. H. Moon, H. M. Jung, S. Lee, Org. Lett. 2008, 10, 945-948; b) J. Moon, M. Jang, S. Lee, J. Org. Chem. 2009, 74, 1403-1406; c) W. Jia, N. Jiao, Org. Lett. 2010, 12, 2000-2003.).

Traditionell sind für die Synthese von Propiolsäuren mehrstufige Verfahren erforderlich, wie die Addition von Alkinen an Formaldehyd und die nachfolgende Oxidation des resultierenden Propagylalkohols (siehe hierzu beispielsweise a) W. Reppe, Liebigs Ann. Chem. 1955, 596, 1-4; b) J. Stohrer, E. Fritz-Langhals, C. Brüninghaus, US7.173.149B2, 2007), die Carbonylierung von instabilen und kommerziell kaum verfügbaren Alkinylhalogenverbindungen (siehe hierzu beispielsweise a) T. Mizuno, H. Alper, Journal of Molecular Catalysis A: Chemical 1997, 123, I-24; b) H. Arzoumanian, F. Cochini, D. Nuel, J. F. Petrignani, N. Rosas, Organometallics 1992, 11, 493-495) oder die Lithiierung von terminalen Alkinen und anschließender Umsetzung mit Chloroformat (siehe hierzu beispielsweise a) J. Tsuji, M. Takahashi, and T. Takahashi, Tetrahedron Lett. 1980, 21, 849; b) E. R. H. Jones, G. H. Whitham, M. C. Whiting, J. Chem. Soc. 1957, 4628-4633; c) N. Satyanarayana, H. Alper, Organometallics 1991, 10, 804-807; d) J. Li, H. Jiang, M. Chen, Synth. Commun. 2001, 31, 199-202; e) Y. Izawa, I. Shimizu, A. Yamamoto, Bull. Chem. Soc. Jpn. 2004, 77, 2033-2045; f) L. Kollár, Modern Carbonylation Reactions, Wiley-VCH, Weinheim, 2008, pp. 276-280.). Darüberhinaus gibt es Verfahren zur oxidativen Carbonylierung von Alkinen mit Kohlenmonoxid. Die Nachteile all dieser Zugangswege ergeben sich aus der Wahl der C1-Bausteine: Formaldehyd und Chloroformat sind giftig und vergleichsweise teuer, Kohlenmonoxid ist ein schwer handhabbares, giftiges Gas.

Kohlendioxid ist in vielerlei Hinsicht ein attraktiverer C1-Baustein zum Aufbau der Carboxylateinheit (a) T. Sakakura, K. Kohon, Chem. Commun. 2009, 1312-1330; b) T. Sakakura, J.-C. Choi, H. Yasuda, Chem. Rev. 2007, 107, 2365-2387; c) N. Eghbali, C.-J. Li, Green Chem. 2007, 9, 213-215; d) H. Arakawa, et al., Chem. Rev. 2001, 101, 953-996.). Es ist als Abfallprodukt aus vielerlei Verbrennungsprozessen kostengünstig in großer Menge verfügbar und einfach handhabbar. Die stoffliche Verwendung von Kohlendioxid ist auch ökologisch vorteilhaft als sie dem durch CO₂ verursachten Treibhauseffekt entgegen wirkt.

Bisher gelingt die Synthese von Propiolsäuren aus Kohlendioxid jedoch nur in Verbindung mit teueren metallorganischen Reagenzien, beispielsweise Alkinylmagnesium-, Alkinylzink- oder Alkinyllithiumsreagenzien (L. Brandsma, Preparative Acetylenic Chemistry, 2nd Ed., Elsevier, Amsterdam, 1998.). Die Carboxylierung solch teurer Verbindungen, deren Synthese starke Metallbasen erfordert, ist ökonomisch unvorteilhaft.

Die Synthese von Propiolsäuren aus Kohlendioxid gelingt auch ausgehend von Natrium-Acetylid (Strauss, Voss, Ber. dt. chem. Ges. 1926, S. 1681-1691). Natrium-Acetylid lässt sich nur durch extrem starke Basen wie z.B. Natriumhydrid oder metallisches Natrium erzeugen. Die Anwesenheit sauerstoffhaltiger Basen wie beispielsweise Natriumhydroxid führt zur Verkohlung oder Selbstentzündung des Reaktionsgemisches, die nur durch Beimengen großer Mengen an Sand vermieden werden kann. Die Umsetzung des Natrium-Acetylids mit Kohlendioxid unter den von Strauss et al. berichteten Bedingungen ist extrem langsam, erst nach drei Wochen wurden ausreichende Umsätze beobachtet. Derart langsame Reaktionen sind für industrielle Anwendungen ungeeignet.

Die optimale Strategie sowohl aus ökonomischer wie auch aus ökologischer Sicht wäre eine einstufige katalytische Carboxylierung von terminalen Alkinen mit Kohlendioxid unter C-H Funktionalisierung zu den entsprechenden Alkincarbonsäuren. Es bestand daher Bedarf an einem Verfahren, das die Umsetzung von terminalen Alkinen mit Kohlendioxid in Gegenwart von schwachen Basen erlaubt.

Kupplungsreaktionen von Alkinen unter C-H Bindungsbruch in Gegenwart von lediglich schwachen Basen, also Verbindungen, deren Basizität nicht ausreicht, um ein terminales Alkin in Abwesenheit eines Katalysators zu deprotonieren, sind bekannt für Palladium / Kupfer vermittelte Kreuzkupplungen mit Arylhalogeniden (Sonogashira-Reaktion) (K. Sonogashira, E.-I. Negishi, Eds. Handbook of Organopalladium Chemistry for Organic Synthesis; Wiley-VCH: New York, 2004; pp 493-529.) oder übergangsmetallvermittelte 1,2-Additionen von Alkinen (D. Boyall, D. E. Frantz, E. M. Carreira, Org. Lett. 2002, 4, 2605-2606.). Es gab jedoch bisher kein Beispiel für ein Verfahren, mit dem terminale Alkine mit einer schwachen Base in Gegenwart eines Übergangsmetalls deprotoniert und unmittelbar im Reaktionsgemisch mit Kohlendioxid in hohen Ausbeuten zu den Propiolsäuren umgesetzt werden können. Die Ursache liegt darin, dass bekannte Carboxylierungskatalysatoren relativ hohe Temperaturen erfordern. Die durch Carboxylierung von Alkinen mit Kohlendioxid in Gegenwart von Kupfersalzen zugänglichen Propiolsäure-Produkte sind aber thermisch so instabil, dass sie unter Extrusion von Kohlendioxid unmittelbar wieder zu den Alkin-Startmaterialien zerfallen, sobald die Zufuhr von Kohlendioxid beendet wird (T. Tsuda, K. Ueda, T. Saegusa, J. C. S. Chem. Comm. 1974, 380-381.).

Saegusa *et al.* zeigten, dass einfache Kupfersalze die reversible Fixierung von Kohlendioxid erlauben. Aufgrund der Reversibilität der Reaktion gelang es Ihnen jedoch nicht, die im Gleichgewicht gebildeten Propiolsäuren zu isolieren (T. Tsuda, K. Ueda, T. Saegusa, J. C. S. Chem. Comm. 1975, 963-964.). Erst als sie Alkylierungsmittel, i.e. 1-Bromhexan, zusetzten und somit die Carbonsäuren kontinuierlich als Ester aus dem Gleichgewicht entfernten, konnten sie zufrieden stellende Umsätze erzielen (Y. Fukue, S. Oi, Y. Inoue, J. C. S. Chem. Comm. 1994, 2091.). Die Verwendung von Halogenverbindungen macht das Gesamtverfahren jedoch unvorteilhaft, ganz besonders wenn nicht die Propiolsäureester, sondern die Propiolsäuren die gewünschten Zielverbindungen sind.

Diese Reversibilität der Insertion von Kohlendioxids ist ein generelles Problem bei Carboxylierungsreaktionen. Nolan *et al.* berichteten kürzlich über die Carboxylierung C≡H-acider Heterocyclen in Gegenwart von Goldkatalysatoren (I. I. F. Boogaerts, S. P. Nolan, J. Am. Chem. Soc. 2010, 132, 8858-8859.). Auch in diesem Fall wurden die resultierenden Carbonsäuren überwiegend in Form der korrespondierenden Ester isoliert.

Im Gegensatz zur Umsetzung von Acetylenen mit Kohlendioxid ist die zuvor beschriebene Reaktion von Metall-Acetyliden mit Kohlendioxid in Abwesenheit jeglicher Protonenquelle irreversibel. Die Voraussetzung dafür ist jedoch, dass die Bedingungen so basisch sind, dass sich kein Acetylen, sondern nur ein Metall-Acetylid bilden könnte.

Der vorliegenden Erfindung liegt die Aufgabe zugrunde, ein Verfahren bereitzustellen, das die Herstellung von Propiolsäuren durch Umsetzung entsprechender terminaler Alkine mit CO₂ in vernünftigen Ausbeuten und mit wirtschaftlich vertretbarem Aufwand erlaubt. Insbesondere sollte das Verfahren gewährleisten, dass das Gleichgewicht der enthalpisch vorteilhaften aber entropisch ungünstigen Carboxylierung von Alkinen weit auf die Seite der Propiolsäuren verschoben werden kann. Nach dem Verfahren sollen Propiolsäuren aus Alkinen und Kohlendioxid in guten Ausbeuten erzeugt und unzersetzt isoliert werden können.

Diese und weitere Aufgaben werden durch das in den Ansprüchen dargelegte und im Folgenden näher erläuterte Verfahren gelöst.

Die Erfindung betrifft ein Verfahren zur Herstellung einer Propiolsäure oder eines Derivates davon durch Umsetzung eines terminalen Alkins mit Kohlendioxid, das dadurch gekennzeichnet ist, dass die Umsetzung in Gegenwart einer Base und einem Kupferkomplex, insbesondere einem Kupfer(I)-Komplex, durchgeführt wird, der wenigstens einen mehrzähnigen Liganden, der wenigstens zwei zur gleichzeitigen Koordination mit Kupfer befähigte Atome oder Atomgruppen aufweist, die unter Stickstoff, Sauerstoff, Schwefel, Phosphor und Carben-Kohlenstoff ausgewählt sind. Dabei weist der Kupferkomplex wenigstens einen weiteren Liganden auf, der unter Phosphinen ausgewählt ist.

Die Erfindung betrifft insbesondere ein Verfahren zur Herstellung einer Propiolsäure oder eines Derivates davon durch Umsetzung eines terminalen Alkins mit Kohlendioxid, das dadurch gekennzeichnet ist, dass die Umsetzung in Gegenwart einer Base und einem Kupferkomplex mit wenigstens einem mehrzähnigen Stickstoffliganden durchgeführt wird, der wenigstens zwei zur Koordination mit Kupfer befähigte Atome oder Atomgruppen aufweist, die unter Stickstoff, Sauerstoff, Schwefel, Phosphor und Carben-Kohlenstoff ausgewählt sind.

Die erfindungsgemäß verwendeten Kupferkomplexe senken die Aktivierungsbarriere für die Einschiebung von Kohlendioxid in die acetylenische C-H-Bindung des terminalen Alkins so stark ab, dass das Carboxylierungs- / Decarboxylierungsgleichgewicht bereits bei geringen Partialdrücken an Kohlendioxid fast vollständig auf der Seite der carboxylierten Produkte, i.e. der Propiolsäuren liegt. Dass dies in so hoher Effizienz gelang, ist insofern überraschend, als solche Kupferkatalysatoren auch die unerwünschte Rückreaktion, die Decarboxylierungsreaktion, gut katalysieren (a) L. J. Gooßen, W. R. Thiel, N. Rodríguez, C. Linder, B. Melzer, Adv. Synth. Catal. 2007, 349, 2241-2246; b) L. J. Gooßen, F. Manjolinho, B. A. Khan, N. Rodríguez, J. Org. Chem. 2009, 74, 2620-2623; c) L. J. Gooßen, N. Rodríguez, C. Linder, P. P. Lange, A. Fromm, ChemCatChem 2010, 2, 430-442.).

Unter "terminalen Alkinen" werden Verbindungen mit wenigstens einer Gruppe -C≡C-H verstanden.

Die terminalen Alkine weisen z. B. die allgemeine Formel X

R^{x}-C≡C-H (X)

auf, worin R^{x} für Wasserstoff, COOR^{x1}, Alkyl, Alkenyl, Cycloalkyl, Heterocycloalkyl, Aryl, Hetaryl oder (R^{x2})₃Si steht und wobei Alkyl und Alkenyl unsubstituiert sind oder einen oder mehrere Substituenten, z. B. 1, 2, 3, 4 oder 5 Substituenten R^{x3} aufweisen und worin Cycloalkyl, Heterocycloalkyl, Aryl und Hetaryl unsubstituiert sind oder einen oder mehrere Substituenten, z. B. 1, 2, 3, 4 oder 5 Substituenten R^{x4} aufweisen, wobei
- R^{x1}: ausgewählt ist unter Wasserstoff, Alkyl, Cycloalkyl, Heterocycloalkyl, Aryl und Hetaryl, wobei die vier zuletzt genannten Reste unsubstituiert sind oder einen oder mehrere, z.B. 1, 2 oder 3, Reste aufweisen, die unter Hydroxy (= OH), Mercapto (= SH), NE¹E², C(O)NE¹E², Halogen, Nitro (= NO₂), Nitroso (= NO), Formyl (= C(=O)H), Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind,
- R^{x2}: ausgewählt ist unter Alkyl, Cycloalkyl, Heterocycloalkyl, Aryl und Hetaryl, wobei die vier zuletzt genannten Reste unsubstituiert sind oder einen oder mehrere, z.B. 1, 2 oder 3, Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind,
- R^{x3}: ausgewählt ist unter Halogen, Cyano, Hydroxy, Mercapto, Alkoxy, COOH, SO₃H, NE¹E², C(O)NE¹E², Acyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl und Hetaryloxycarbonyl, wobei die cyclischen Gruppen in den zwölf zuletzt genannten Resten unsubstituiert sind oder einen oder mehrere, z.B. 1,2 oder 3, Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl, ausgewählt sind,
- R^{x4}: ausgewählt ist unter Halogen, Cyano, Nitro, Hydroxy, Merkapto, Alkoxy, COOH, SO₃H, NE¹E², C(O)NE¹E², Alkyl, Halogenalkyl, Acyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl und Hetaryloxycarbonyl, wobei die cyclischen Gruppen in den zwölf zuletzt genannten Resten unsubstituiert sind oder einen oder mehrere, z.B. 1, 2 oder 3, Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind,

worin E¹ und E² gleiche oder verschiedene Reste, ausgewählt unter Wasserstoff, Alkyl, Cycloalkyl, oder Aryl bedeuten oder E¹ und E² gemeinsam mit dem Stickstoffatom, an das sie gebunden sind, für gesättigtes Stickstoffheterocyclyl stehen, der unsubstistuiert ist oder einen oder mehrere Alkylgruppen als Substituenten aufweist.

Die bei dem erfindungsgemäßen Verfahren erhaltenen Propiolsäure weisen z. B. die folgende allgemeine Formel XI

Rx-C≡C-COOH (XI)

auf, worin R^{x} die zuvor für Formel X genannten und insbesondere die im Folgenden genannten Bedeutungen aufweist. Sofern Acetylen als terminales Alkin eingesetzt wird (R^{x} = H), kann in Abhängigkeit von der Reaktionsführung auch Acetylendicarbonsäure hergestellt werden.

Bevorzugt werden im erfindungsgemäßen Verfahren solche terminalen Alkine der Formel X umgesetzt, worin R^{x} für Wasserstoff, Alkyl, Cycloalkyl oder Phenyl steht, wobei Alkyl unsubstituiert ist oder 1 oder 2 Reste R^{x3} trägt die vorzugsweise unter Alkoxy, Cycloalkyl und Phenyl ausgewählt sind und wobei Phenyl und Cycloalkyl unsubstituiert sind oder 1, 2 oder 3 Reste R^{x4} tragen, die vorzugsweise unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind. In einer besonders bevorzugten Ausführungsform der Erfindung wird Acetylen als terminales Alkin eingesetzt, also ein terminales Alkin, worin R^{x} für Wasserstoff steht.

Hier und im Folgenden stellen die im Zusammenhang mit den Substiuenten genannten Bedeutungen Halogen, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Acyl, Alkoxycarbonyl, Cycloalkyl, Heterocycloalkyl, Aryl, Hetaryl, Cycloalkoxy, Heterocycloalkoxy, Aryloxy, Hetaryloxy, Arylthio, Hetarylthio, Cycloalkoxycarbonyl, Heterocycloalkoxycarbonyl, Aryloxycarbonyl und Hetaryloxycarbonyl, Sammelbegriffe für Gruppen von Substituenten dar. In Zusammenhang mit den Substituenten gibt das Präfix Cₙ-Cₘ den Bereich für die jeweils mögliche Anzahl an Kohlenstoffatomen an, die ein solcher Substituent aufweisen kann.

Halogen steht für Fluor, Chlor, Brom und lod, bevorzugt für Fluor, Chlor und Brom.

Der Ausdruck "Alkyl" umfasst geradkettige und verzweigte Alkylgruppen mit in der Regel 1 bis 20 C-Atomen (C₁-C₂₀-Alkyl), häufig 1 bis 12 C-Atomen (C₁-C₁₂-Alkyl) und insbesondere 1 bis 8 C-Atomen (C₁-C₈-Alkyl). Vorzugsweise handelt es sich dabei um geradkettige oder verzweigte C₁-C₁₂-Alkyl- und besonders bevorzugt um C₁-C₈-Alkylgruppen oder C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethyl-butyl, 2,2-Dimethylbutyl, 3,3-Dimethylbutyl, 1,1,2-Trimethylpropyl, 1,2,2-Trimethyl-propyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl und Decyl.

Der Ausdruck "Halogenalkyl" umfasst geradkettige und verzweigte Alkylgruppen mit in der Regel 1 bis 20 C-Atomen (C₁-C₂₀-Halogenalkyl), häufig 1 bis 12 C-Atomen (C₁-C₁₂-Halogenalkyl) und insbesondere 1 bis 8 C-Atomen (C₁-C₈-Halogenalkyl) oder 1 bis 4 C-Atomen (C₁-C₈-Halogenalkyl), worin wenigstens eines, z.B. 1, 2, 3, 4 oder 5 der Wasserstoffatome durch Halogenatome, insbesondere durch Fluoratome ersetzt sind. Beispiele für Halogenalkylgruppen sind Fluormethyl, Difluormethyl, Trifluormethyl, 1-Fluorethyl, 2-Fluorethyl, 1,1-Difluorethyl, 2,2-Difluorethyl, 1,2-Difluorethyl, 2,2,2-Trifluorethyl, 1,1,2,2-Tetrafluorethyl, Pentafluorethyl, 1-Fluorpropyl, 2-Fluorpropyl, 3-Fluorpropyl, 2,3-Difluorpropyl, 1,1-Difluorpropyl, 1,2-Difluorpropyl, 2,2-Difluorpropyl, 3,3-Difluorpropyl, 3,3,3-Trifluorpropyl, 2,2,3,3,3-Pentafluorpropyl, Heptafluorpropyl, 2-Fluor-2-propyl, 1-Fluor-2-proly, 1,1-Difluor-2-propyl, 1,1,1-Trifluor-2-propyl und Heptafl uor-2-propyl.

Der Ausdruck "Alkenyl" umfasst geradkettige und verzweigte Kohlenwasserstoffgruppen mit wenigstens einer ethylenischen Unsättigung. Vorzugsweise handelt es sich dabei um geradkettige oder verzweigte C₂-C₂₀-Alkenyl-, bevorzugterweise C₂-C₁₂-Alkenyl-, besonders bevorzugt C₂-C₈-Alkenylgruppen
Der Ausdruck "Cycloalkyl" umfasst vorzugsweise C₅-C₇-Cycloalkylgruppen, wie Cyclopentyl, Cyclohexyl oder Cycloheptyl.

Der Ausdruck "Heterocycloalkyl" umfasst gesättigte, cycloaliphatische Gruppen mit im Allgemeinen 4 bis 7, vorzugsweise 5 oder 6 Ringatomen, in denen 1, 2, 3 oder 4 der Ringkohlenstoffatome durch Heteroatome, vorzugsweise ausgewählt aus den Elementen Sauerstoff, Stickstoff und Schwefel, ersetzt sind und die gegebenenfalls substituiert sein können. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Der Ausdruck "Aryl" umfasst vorzugsweise C₆-C₁₄-Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl.

Der Ausdruck "Hetaryl" umfasst heterocycloaromatische Gruppen, die aus einem oder zwei oder drei anellierten 5- oder 6-gliedrigen aromatischen Ringen aufgebaut sind, worin in wenigstens einem Ring 1, 2, 3 oder 4 der Ringkohlenstoffatome durch ein Heteroatom, vorzugsweise ausgewählt aus den Elementen Sauerstoff, Stickstoff und Schwefel, ersetzt sind, vorzugsweise die Gruppen Pyridyl, Chinolinyl, Acridinyl, Pyridazinyl, Pyrimidinyl, Pyrazinyl, Triazinyl, Tetrazinyl, Pyrrolyl, Pyrazolyl, Isoxazolyl, Imidazolyl, Oxazolyl, Thiazolyl, Thiophenyl, Furanyl.

Die obigen Erläuterungen zu den Ausdrücken "Alkyl", "Halogenalkyl", "Cycloalkyl", "Aryl", "Heterocycloalkyl" und "Hetaryl" gelten entsprechend für die Ausdrücke "Alkoxy", "Halogenalkoxy", "Alkylthio", "Halogenalkylthio", "Alkylcarbonyl", "Alkylcarbonyloxy", "Halogenalkylcarbonyl", "Halogenalkylcarbonyloxy", "Aryloxy", "Arylthio", "Hetaryloxy", "Heterocycloalkoxy", "Hetarylthio", "Alkoxy(carbonyl)", "Cycloalkoxy(carbonyl)", "Aryloxy(carbonyl)", "Heterocycloalkoxy(carbonyl)" und "Hetaryloxy(carbonyl)". Dabei steht "Alkoxy" für einen über ein Sauerstoffatom gebundenen Alkylrest, wie zuvor definiert. "Halogenalkoxy", "Cycloalkoxy", "Heterocycloalkoxy", "Aryloxy" und "Hetaryloxy" stehen dementsprechend für eine über ein Sauerstoffatom gebundene Halogenalkyl-, Cycloalkyl-, Heterocycloalkyl-, Aryl- bzw. Hetarylgruppe. "Alkylthio" für einen über ein Schwefelatom gebundenen Alkylrest, wie zuvor definiert. "Halogenalkylthio", "Cycloalkylthio", "Heterocycloalkylthio", "Arylylthio" und "Hetarylylthio" stehen dementsprechend für eine über ein Schwefelatom gebundene Halogenalkyl-, Cycloalkyl-, Heterocycloalkyl-, Aryl- bzw. Hetarylgruppe

Der Ausdruck "Acyl" steht im Sinne der vorliegenden Erfindung für die Formylgruppe oder Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Gruppe NE¹E² steht vorzugsweise für N,N-Dimethylamino, N,N-Diethylamino, N,N-Dipropylamino, N-Methyl-N-ethylamino, N-Methyl-N-propylamino, N-Methyl-N-isopropylamino, N-Methyl-N-butylamino, N-Methyl-N-tert.-butylamino, N-Methyl-N-cyclohexylamino, N-Methyl-N-phenylamino, N,N-Diisopropylamino, N,N-Di-n-butylamino, N,N-Di-tert-butylamino, N,N-Dicyclohexylamino, N,N-Diphenylamino, 4-Morpholinyl, 1-Piperidinyl, 1-Pyrrolidinyl oder 4-Methyl-1-piperazinyl.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind orthokondensierte Ringsysteme.

Im erfindungsgemäßen Verfahren werden terminale Alkine mit Kohlendioxid gemäß dem allgemeinen Schema 1 in Gegenwart von Kupferkomplexen und Basen zu Propiolsäuren oder Derivaten davon umgesetzt. Derivate sind insbesondere Metallsalze und Ester der Propiolsäuren. Bevorzugt wird die Umsetzung so durchgeführt, dass man nach Aufarbeitung die freie Propiolsäure erhält.

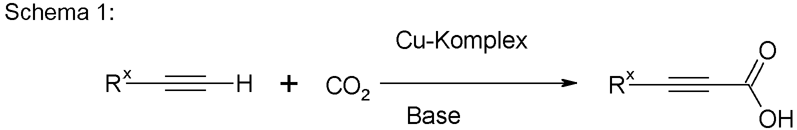

Für R^{x} = H, also wenn Acetylen als Substrat eingesetzt wird, kann gemäß Schema 2 wahlweise auf nur einer oder auch auf beiden Seiten carboxyliert werden, wobei sich dann die Acetylendicarbonsäure bildet. Diese Reaktion, die auch in Beispiel 41 näher erläutert wird, ist insofern besonders überraschend, da Acetylen mit Kupfersalzen bekanntermaßen stabile Komplexe bildet, und sich in seiner Reaktivität gegenüber Übergangsmetallen oftmals deutlich von seinen längerkettigen Derivaten unterscheidet.

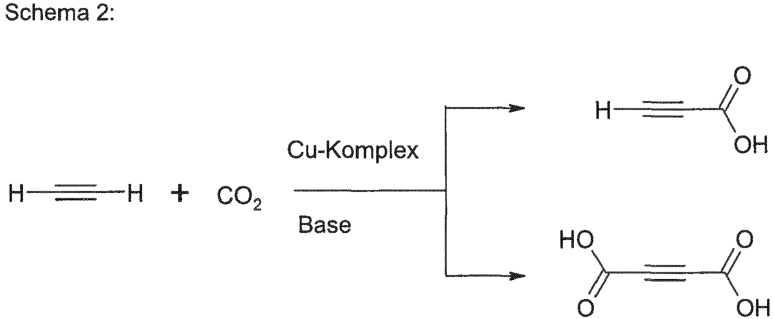

Im erfindungsgemäßen Verfahren dienen Kupferkomplexe, insbesondere Kupfer(I)-Komplexe, mit wenigstens einem mehrzähnigen Liganden als Katalysatoren. Bei den erfindungsgemäß eingesetzten Kupferkomplexen handelt es sich in der Regel um Chelatkomplexe des mehrzähnigen Liganden mit dem Kupferatom, der einen oder mehrere weitere Liganden aufweist.

Unter "mehrzähniger Ligand" wird eine Verbindung verstanden, die gleichzeitig über wenigstens zwei Donoratome oder Donoratomgruppen eine koordinative Bindung zum Kupferatom ausbilden kann. Solche Donoratome können Heteroatome wie Schwefel, Sauerstoff oder Stickstoff sein. Beispiele für Phosphoratome, die als Donoratome fungieren können, sind "dreibindige" Phosphoratome, die in Form von Phosphingruppen oder Phosphonitgruppen im Liganden vorliegen. Beispiele für Schwefelatome, die als Donoratome fungieren können, sind vor allem ein- und zweibindige Schwefelatome, die beispielsweise als Merkaptangruppen, als Thioethergruppe, als Thiocarbonylgruppen Thioisocyanatgruppen im Liganden vorliegen. Beispiele für Sauerstoffatome, die als Donoratome fungieren können, sind vor allem ein- und zweibindige Sauerstoffatome, die beispielsweise als Hydroxylgruppen, als Carbonylgruppen, Carboxylatgruppenen oder als Oximgruppen im Liganden vorliegen. Beispiele für Stickstoffatome, die als Donoratome fungieren können, sind vor allem ein-bindige, zweibindige oder dreibindige Stickstoffatome, die beispielsweise als primäre, sekundäre oder tertiäre Aminogruppen, als Hydroxylaminogruppen, als Iminogruppen, einschließlich Oximgruppen, oder als Nitrengruppen im Liganden vorliegen. Donoratom kann auch ein Kohlenstoffatom sein, die als zweibindinger Kohlenstoff, d.h. als carbenoid gebundener Kohlenstoff (Carben-Kohlenstoff) m Liganden vorliegen. Bevorzugte mehrzähnige Liganden sind insbesondere solche, die wenigstens ein und insbesondere wenigstens zwei Stickstoffatome als Donoratome aufweisen.

Im erfindungsgemäßen Verfahren werden insbesondere Kupfer(I)-Komplexe, mit wenigstens einem mehrzähnigen Stickstoffliganden, der wenigstens zwei zur Koordination mit Kupferatomen befähigte Stickstoffatome aufweist, als Katalysatoren eingesetzt. Bei den erfindungsgemäß eingesetzten Kupferkomplexen mit mehrzähnigen Stickstofflig- and handelt es sich in der Regel um Chelatkomplexe des mehrzähnigen Stickstoffliganden mit dem Kupferatom, der einen oder mehrere weitere Liganden aufweist.

Unter "mehrzähniger Stickstoffligand" wird eine Verbindung verstanden, die über wenigstens zwei Stickstoff-Donoratome eine koordinative Bindung zum Kupferatom ausbilden kann. Bevorzugt sind zweizähnige Stickstoffliganden, d.h. Liganden, die genau zwei Stickstoffdonoratome aufweisen, welche jeweils eine koordinative Bindung zum Kupferatom ausbilden können. Derartige Liganden werden im Folgenden auch als (N,N)-Ligand bezeichnet. In diesen Liganden liegt der Stickstoff vorzugsweise in Form von Alkylamin-, Cyclolkylamin-, Heterocycloalkylamin-, Arylamin-, Heteroarylamin-, Alkylimin-, Cyclolkylimin-, Heterocycloalkylimin-, Arylimin- oder Heteroarylimin-Gruppe vor, insbesondere in Form einer Heterocyclylimin oder Heteroarylimin-Gruppe, worin die Iminogruppe Bestandteil der Heterocyclylimin- bzw. Heteroarylimingruppe ist, z.B. in Form einer Heteroarylimingruppe, ausgewählt unter Pyridin-, Pyrimidin-, Pyrazin-, Pyridazin-, Pyrrol-, 1H-Indol-, Imidazol-, Oxazol-, Thiazol- und Pyrazol-Gruppen, oder in Form einer Heterocycloalkylimingruppe, ausgewählt unter 3,4,5,6-Tetrahydropyridin-, 1,2,5,6-Tetrahydropyrimidin-, 1,4,5,6-Tetrahydropyrimidin-, 1,2,3,6-Tetrahydropyrazin-, 3,4,5,6-Tetrahydropyridazin-, Pyrrolin-, 3H-Indol-, Imidazolin-, Oxazolin-, Thiazolin- und 4,5-Dihydopyrazol-Gruppen Die vorgenannten cyclischen Gruppen sind ihrerseits unsubstituiert oder weisen einen oder mehrere, z.B. 1, 2, 3 oder 4, der im Folgenden näher erläuterten Substituenten R^{s} auf.

Neben dem mehrzähnigen Stickstoffliganden weist der Kupferkomplex wenigstens einen weiteren, unter Phosphinen ausgewählten Liganden auf. Sämtliche Liganden sind vorzugsweise ungeladen.

Bevorzugt werden Kupfer(I)-Komplexe eingesetzt, d.h. Kupfer liegt in der Oxidationsstufe +I vor. Das zur Ladungsneutralität erforderliche Gegenion ist ein beliebiges Anion, das z. B. ausgewählt ist unter Halogeniden, wie I⁻, Br, Cl⁻, F⁻, (Hydrogen)carbonat [HCO₃⁻, CO₃²⁻], (Hydrogen)phosphaten [PO₄³⁻, HPO₄²⁻, H₂PO₄⁻], Carboxylaten, wie Formiat, Acetat, Propionat, Benzoat; Hydroxid [OH-], Oxid [O²-], Alkoxiden, wie Methanolat, Ethanolat; Phenolaten, Nitrat [NO₃⁻], (Hydrogen)sulfat [SO₄⁻, HSO₄⁻], komlexen Anionen, wie BF₄⁻, PF₆⁻, AsF₆⁻, SbF₆⁻, BPh₄⁻; Sulfonaten, wie Tosylat, Trifluormethansulfonat und Methylsulfonat.

Vorzugsweise weist der mehrzähnige Stickstoffligand ein Gerüst einer der Formel I auf, 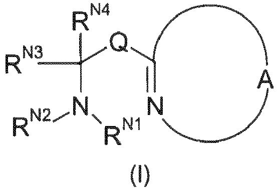
- A: zusammen mit dem Fragment C=N, an das es gebunden ist, einen 5- bis 7-gliedrigen heterocyclischen Ring, insbesondere einen 5- oder 6-gliedrigen heteroaromatischen Ring bildet, der mit einem, zwei oder drei weiteren Ringen anelliert sein kann;
- Q: für eine chemische Bindung oder eine verbrückende Gruppe mit einem, zwei oder drei Atomen steht; wobei die chemische Bindung oder verbrückende Gruppe teilweise oder vollständig Bestandteil eines oder mehrer Ringe sein kann, wobei im Falle einer cyclischen Gruppen Q, diese mit dem Ring A annelliert sein kann;
- R^{N1}: für Wasserstoff steht oder
- R^{N1}: mit R^{N4} eine chemische Bindung bildet,
- R^{N2}: für Alkyl, Cycloalkyl oder Aryl steht
- R^{N3}: für Wasserstoff, Alkyl, Cycloalkyl oder Aryl steht, wobei Alkyl unsubstituiert ist oder einen Rest aus der Gruppe Cycloalkyl oder Aryl trägt;
- R^{N2}: und R^{N3} gemeinsam mit den Atomen, an die sie gebunden sind, einen 5- bis 7-gliedrigen heterocyclischen Ring, insbesondere einen 5- oder 6-gliedrigen heteroaromatischen Ring bilden, der mit einem, zwei oder drei weiteren Ringen, insbesondere einer cyclischen Gruppe Q anelliert sein kann; und
- R^{N4}: für Wasserstoff steht oder abwesend ist oder mit R^{N1} eine chemische Bindung bildet.

Bevorzugte Gerüste der Formel I sind solche, in denen R^{N2} und R^{N3} gemeinsam mit den Atomen, an die sie gebunden sind, einen 5- bis 7-gliedrigen heterocyclischen Ring, insbesondere einen 5- oder 6-gliedrigen heteroaromatischen Ring bilden, der mit einem, zwei oder drei weiteren Ringen, insbesondere einer cyclischen Gruppe Q anelliert sein kann.

Dementsprechend weist der mehrzähnige Stickstoffligand vorzugsweise ein Gerüst einer der Formel II auf, 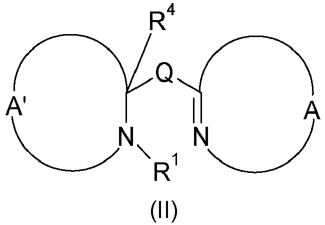 worin A, Q, R^{N1} und R^{N4} die zuvor genannten Bedeutungen aufweisen und worin A', gemeinsam mit dem Fragment -NR^{N1}-CR^{N4}-, an das es gebunden ist, einen 5- bis 7-gliedrigen heterocyclischen Ring, insbesondere einen 5- oder 6-gliedrigen heteroaromatischen Ring bildet, der mit einem, zwei oder drei weiteren Ringen anelliert sein kann.

Bevorzugte Gerüste der Formeln I und II sind solche, in denen R^{N1} mit R^{N4} eine chemische Bindung bildet.

Bevorzugte Liganden sind solche, die ein Gerüst der Formel I oder II aufweisen, in denen der Ring A bzw. einer der Ringe A für 2-Pyridyl bzw. , wenn A mit Q anelliert ist, für eine b-Pyridino-Gruppe steht.

Vorzugsweise steht Q in den Formeln I und I für eine chemische Bindung oder einen 6-gliedrigen Carbocyclus, der mit der Gruppe A ortho-anelliert ist.

Besonders bevorzugte Gerüste der Formel I sind insbesondere 2,2'-Bipyridin (A steht für 2-Pyridyl und Q ist eine chemische Bindung) und 1,10-Phenantrolin (A steht für b-Pyrido und Q ist Benzolring, der mit den Pyridogruppen ortho-anelliert ist).

Beispiele für bevorzugte Gerüste der Formeln I bzw. II sind die im Folgenden exemplarisch genannten Strukturen III.1 bis III.9:

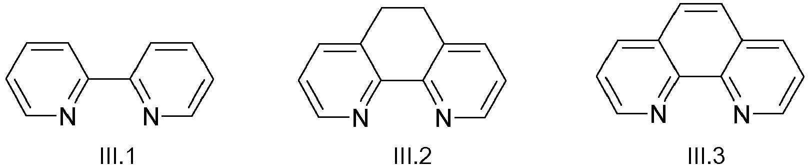

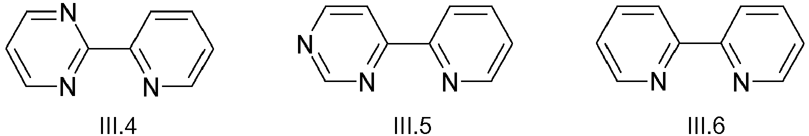

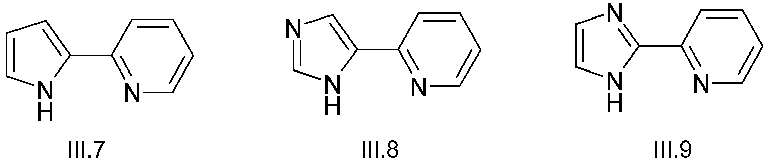

Hierunter sind solche Liganden bevorzugt, die ein Gerüst der Formeln III.1 oder III.2 und insbesondere ein Gerüst der Formel III.3 aufweisen.

Die Gerüste der Formeln I, II und III.1 bis III.9 können einen oder mehrere, z.B. 1, 2, 3 oder 4, Substituenten R^{s} aufweisen, die z.B. ausgewählt sind unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Arylthio, Hetarylthio, Cycloalkoxycarbonyl, Heterocycloalkoxycarbonyl, Aryloxycarbonyl und Hetaryloxycarbonyl, wobei die cyclischen Gruppen in den vierzehn zuletzt genannten Resten unsubstituiert sind oder einen oder mehrere Reste aufweisen können, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl, Hetaryloxycarbonyl ausgewählt sind, und wobei E¹ und E² die zuvor genannten Bedeutungen aufweisen.

Bevorzugte Substituenten R^{s} an den Gerüsten der Formeln I, II und III.1 bis III.9 sind Halogen, CN, NO₂, Alkyl, Alkoxy, Alkylthio, Acyl, Cycloalkyl, Cycloalkoxy, Aryl, Aryloxy und Arylthio, insbesondere unter C₁-C₈-Alkyl, C₁-C₈-Alkoxy, C₁-C₈-Alkylthio, Cyclohexyl, Cyclohexyloxy, Phenyl, Phenoxy und Phenylthio, wobei Aryl, Aryloxy, Arylthio bzw. Phenyl, Phenoxy und Phenylthio unsubstituiert sind oder 1 oder 2 der zuvor genannten Substituenten tragen können, die insbesondere unter Halogen, Alkyl, Halogenalkyl, Alkoxy und Halogenalkoxy ausgewählt sind.

Ganz besonders bevorzugt werden als mehrzähnige Liganden 1,10-Phenantrolinderivate der Formel IV eingesetzt, 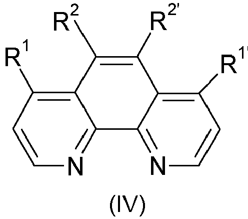 worin
R¹ und R^{1'} unabhängig voneinander für Wasserstoff, Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Arylthio, Hetarylthio, Cycloalkoxycarbonyl, Heterocycloalkoxycarbonyl, Aryloxycarbonyl oder Hetaryloxycarbonyl stehen, worin die cyclischen Gruppen in den vierzehn zuletzt genannten Resten unsubstituiert sind oder einen oder mehrere Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl, Hetaryloxycarbonyl ausgewählt sind, worin
E¹ und E² gleiche oder verschiedene Reste, ausgewählt unter Wasserstoff, Alkyl, Cycloalky und Aryl, bedeuten oder E¹ und E² gemeinsam mit dem Stickstoffatom, an das sie gebunden sind, für gesättigtes Stickstoffheterocyclyl stehen, der unsubstistuiert ist oder einen oder mehrere Alkylgruppen als Substituenten aufweist, und

Vorzugsweise ist wenigstens oder sind insbesondere beide Reste R¹ und R^{1'} von Wasserstoff verschieden. Vorzugsweise sind beide Reste R¹ und R^{1'} ausgewählt unter Hydroxy, Mercapto, Alkyl, Alkoxy, Alkylthio, Cycloalkyl, Cycloalkoxy, Aryl, Aryloxy und A-rylthio, insbesondere unter C₁-C₈-Alkyl, C₁-C₈-Alkoxy, C₁-C₈-Alkylthio, Cyclohexyl, Cyclohexyloxy, Phenyl, Phenoxy und Phenylthio, wobei Aryl, Aryloxy, Arylthio bzw. Phenyl, Phenoxy und Phenylthio unsubstituiert sind oder 1 oder 2 der zuvor genannten Reste tragen können, die insbesondere unter Halogen, Alkyl, Halogenalkyl, Alkoxy und Halogenalkoxy ausgewählt sind.

Insbesondere stehen beide Reste R¹ und R^{1'} für Aryl, insbesondere Phenyl, wobei Aryl bzw. Phenyl unsubstituiert sind oder 1 oder 2 Reste tragen können, die unter Halogen, Alkyl, Halogenalkyl, Alkoxy und Halogenalkoxy ausgewählt sind.

Vorzugsweise stehen die Reste R² und R^{2'} unabhängig voneinander für Wasserstoff, Alkyl, Halogen, Nitro, Acyl oder Cyano. Insbesondere stehen R² und R^{2'} für Wasserstoff.

Besonders bevorzugt sind solche Liganden der Formel IV, worin R¹ und R^{1'} für Aryl, insbesondere Phenyl, stehen, wobei Aryl bzw. Phenyl unsubstituiert sind oder 1 oder 2 Reste tragen können, die unter Halogen, Alkyl, Halogenalkyl, Alkoxy und Halogenalkoxy ausgewählt sind und wobei Aryl bzw. Phenyl insbesondere unsubstituiert sind und R² und R^{2'} für Wasserstoff stehen.

Ein ganz besonders bevorzugter mehrzähniger Stickstoffligand ist 4,7-Diphenyl-1,10-phenanthrolin.

Der Kupferkomplex weist wenigstens einen weiteren Liganden, ausgewählt unter Phosphinen, auf. Davon sind Phosphinliganden besonders bevorzugt, die 1 Phosphoratom aufweisen.

Die Verwendung von Kupfer(I)-Komplexen, die einen Stickstoffliganden der Formel IV und wenigstens einen Phosphinliganden umfassen, wobei im Stickstoffliganden der Formel IV die Reste R¹ und R^{1'} von Wasserstoff verschieden sind (im Folgenden als Liganden der Formel IV' bezeichnet), als Carboxylierungskatalysator für terminale Alkine sind neu und ebenfalls Gegenstand der vorliegenden Erfindung, auch wenn einige dieser Kupferkomplexe als solche bekannt sind, wie z.B. (4,7-Diphenyl-1,10-phenantrolin)-bis-(triphenylphosphin)-Kupfer(I)-Tetrafluoroborat (Che, Guang Bo et al., Acta Crystallographica, section E: Structures Reports Online (2006); E62(1), m85-m86-abstract).

Bevorzugte Kupferkomplexe entsprechen somit der Formel V

(N,N)CuLₙ⁺ X- (V)

worin (N,N) für einen zweizähnigen N,N-Liganden, insbesondere einen Liganden der Formel I und speziell für einen Liganden der Formel IV steht, L für einen Phosphinliganden steht, n für eine ganze Zahl von 1 bis 3 steht, und X⁻ für ein Äquivalent eines Anions, z.B. eines der der vorstehenden Anionen, steht.

Vorzugsweise ist der Phosphinligand ausgewählt ist unter Verbindungen der Formel VI

PR^{a}R^{b}R^{c} (VI)

worin R^{a}, R^{b} und R^{c} unabhängig voneinander für Alkyl, Cycloalkyl, Heterocycloalkyl, Aryl oder Hetaryl stehen, wobei die Alkylreste 1, 2, 3, 4 oder 5 Substituenten, ausgewählt unter Cycloalkyl, Heterocycloalkyl, Aryl, Hetaryl, Alkoxy, Cycloalkoxy, Heterocycloalkoxy, Aryloxy, Hetaryloxy, COOH, Carboxylat, SO₃H, Sulfonat, NE¹E², Halogen, Nitro, Acyl oder Cyano aufweisen können, worin E¹ und E² die zuvor genannten Bedeutungen aufweisen, und wobei die Cycloalkyl-, Heterocycloalkyl-, Aryl- und Hetarylreste 1, 2, 3, 4 oder 5 Substituenten aufweisen können, die ausgewählt sind unter Alkyl und den zuvor für die Alkylreste R^{a}, R^{b} und R^{c} genannten Substituenten, wobei R^{a} und R^{b} zusammen mit dem Phosphoratom, an das sie gebunden sind, auch für einen 5- bis 8-gliedrigen P-Heterocyclus stehen können, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl und den zuvor für die Alkylreste R^{a}, R^{b} und R^{c} genannten Substituenten.

Insbesondere ist der wenigstens eine weitere Ligand unter Triarylphosphinen ausgewählt. Tri(*p*-fluorphenyl)phosphin ist besonders bevorzugt.

Das erfindungsgemäße Verfahren kann in Substanz oder in Gegenwart eines Lösungsmittels und in Substanz durchgeführt werden, wobei letzteres bevorzugt ist.

Als Lösungsmittel eignen sich beispielsweise
- aliphatische Kohlenwasserstoffe, wie Pentan, Hexan, Heptan, Octan oder Cyclohexan;
- aromatische Kohlenwasserstoffe, wie Benzol, Toluol, Xylole, Ethylbenzol oder Mesitylen;
- Amide, wie Dimethylformamid, Diethylformamid, *N*-Methylpyrrolidon, N-Ethylpyrrolidon oder Dimethylacetamid;
- Harnstoffe wie Tetramethylharnstoff, N,N-Dimethylimidazolinon (DMI) und N,N-Dimethylpropylenharnstoff (DMPU);
- Nitrile, wie Acetonitril oder Propionitril;
- Sulfoxide, wie Dimethylsulfoxid;
- Sulfone, wie Sulfolan;
- Alkohole, wie Methanol, Ethanol, Propanol oder Isopropanol;
- Ester, wie Methylacetat, Ethylacetat, *t*-Butylacetat
- Carbonate wie Diethylcarbonat, Ethylencarbonat und Propylencarbonat; und
- Ether, wie Dioxan, Tetrahydrofuran, Diethylether, Dibutylether, Methyl-*t-*butylether, Diisopropylether oder Diethylenglycoldimethylether.

Wahlweise kann auch eine Kombination mehrerer Lösungsmittel eingesetzt werden.

Bevorzugt werden als Lösungsmittel aromatische Kohlenwasserstoffe, Amide, Harnstoffe, Ester und Ether und Gemische davon eingesetzt. Besonders bevorzugt werden Lösungsmittel und Lösungsmittelgemische verwendet, die Amide und/oder Harnstoffe enthalten, wobei der Anteil der Amide bzw. Harnstoffe vorzugsweise wenigstens 50 Vol.-% des zur Umsetzung eingesetzten Lösungsmittels ausmachen. Ganz besonders bevorzugt werden Lösungsmittel und Lösungsmittelgemische verwendet, die Amide aus der Reihe Dimethylformamid, Diethylformamid, *N*-Methylpyrrolidon, Dimethylacetamid enthalten, wobei der Anteil dieser Amide vorzugsweise wenigstens 50 Vol.-% des zur Umsetzung eingesetzten Lösungsmittels ausmachen.

Die Kupferkomplexe können im erfindungsgemäßen Verfahren wahlweise in präformierter Form eingesetzt werden und direkt im Reaktionsgemisch aus geeigneten Kupfervorstufen und den entsprechenden Liganden erzeugt werden. Bevorzugt werden präformierte Kupferkomplexe eingesetzt.

Beim erfindungsgemäßen Verfahren wird im Allgemeinen eine Katalysatormenge in substöchiometrischer Menge eingesetzt, wobei die Katalysatormenge typischerweise nicht mehr als 50 mol%, häufig nicht mehr als 20 mol% und insbesondere nicht mehr als 10 mol% oder nicht mehr als 5 mol%, bezogen auf das Alkin beträgt. Beim erfindungsgemäßen Verfahren wird im Allgemeinen eine Katalysatormenge von 0,001 bis 50 mol%, häufig 0,001 mol% bis 20 mol% und insbesondere 0,005 bis 5 mol%, bezogen auf das Alkin eingesetzt. Vorzugsweise wird eine Katalysatormenge von 0,01 bis 2 mol% und besonders bevorzugt 0,01 mol% bis 1 mol% eingesetzt. Alle Angaben zu Katalysatormengen sind als Cu gerechnet und auf die Menge an Alkin bezogen.

Das erfindungsgemäße Verfahren wird im Allgemeinen bei Temperaturen im Bereich von -20 °C bis 200 °C, vorzugsweise im Bereich von20 °C bis 80 °C und besonders bevorzugt im Bereich von35 °C bis 50 °C durchgeführt. Überraschend erwiesen sich Temperaturen unterhalb von 60 °C als besonders vorteilhaft. Bei höheren Temperaturen, wie sie andere Verfahren verwenden, wird mit diesen Katalysatoren die Rückreaktion (Decarboxylierung) bevorzugt.

Im erfindungsgemäßen Verfahren wird das Kohlendioxid bevorzugt in gasförmigen Zustand eingesetzt. Besonders bevorzugt werden CO₂ Partialdrücke von 0,1 bis 20 bar und ganz besonders bevorzugt von 1-10 bar eingesetzt.

Als Basen werden im erfindungsgemäßen Verfahren vorzugsweise solche Basen eingesetzt, deren korrespondierende Säure signifikant stärker ist als das eingesetzte terminale Alkin, so dass das terminale Alkin durch die Base nicht quantitativ deprotoniert wird. Vorzugsweise werden solche Basen eingesetzt, deren korrespondierende Säureeinen pKs-Wert aufweist, der wenigstens 3 pK-Einheiten, insbesondere wenigstens 5 pK-Einheiten und speziell wenigstens 8 pK-Einheiten, z.B. 3 bis 22 pK-Einheiten, insbesondere 5 bis 22 pK-Einheiten, speziell 8 bis 20 pK-Einheiten unterhalb des pKs-Wertes des eingesetzten terminalen Alkins liegt. Insbesondere bevorzugt sind Basen, deren korrespondierende Säure eine pKₛ-Wert im Bereich von 4 bis 20 und insbesondere im Bereich von 5 bis 15 aufweist. Die hier angegebenen pKₛ-Werte sind die negativ dekadischen Logarithmen der bei 25°C bestimmten Säurekonstanten in Wasser bzw. extrapoliert auf Wasser. Bevorzugte Basen sind Oxo-Basen, d.h. das basische Zentrum, an welches das abstrahierte Proton bindet ist ein Sauerstoffatom. Insbesondere werden als Basen anorganische Salze eingesetzt. Bevorzugt werden anorganische Basen verwendet, die aus der Reihe der Alkali- und Erdalkalihydroxide,-carbonate, -bicarbonate, -oxide, -phosphate, -hydrogenphosphate, -fluoride und - carboxylate, z.B. -acetate ausgewählt sind. Besonders bevorzugt werden Basen verwendet, die aus der Gruppe der Alkali- und Erdalkaliphosphate, der Alkali und Erdalkalicarbonate und Alkali und Erdalkalicarboxylate, z.B. der Alkali- und Erdalkaliacetate ausgewählt sind. Ganz besonders bevorzugt werden Alkalimetallcarbonate wie Natriumcarbonat, Kaliumcarbonatoder Cäsiumcarbonat verwendet. Ebenfalls besonders bevorzugt werden Alkalimetallphosphate wie Kaliumphosphat verwendet. Die Base wird in der Regel in wenigstens stöchiometrischer Menge, bezogen auf das eingesetzte terminale Alkin, eingesetzt, vorzugsweise in einem stöchiometrischen Überschuss, z.B. in einer Menge von 1,1 Mol bis 10 Mol, speziell 1,1 bis 3 Mol pro mol terminalem Alkin.

Zur Isolierung der erfindungsgemäß hergestellten Produkte wird das Reaktionsgemisch nach Beendigung der Reaktion vorzugsweise destillativ und/oder durch Extraktion oder Kristallisation aufgearbeitet. Die Produkte werden dabei wahlweise als Carboxylatsalze oder als freie Säuren isoliert.

Alternativ können die primär erhaltenen Carbonsäuresalze unmittelbar im Reaktionsgemisch durch Zusatz von Alkylierungsmitteln aus der Reihe Alkylhalogenide, Alkylsulfonate und Dialkylsulfate in die entsprechenden Alkylester überführt werden.

Die Erfindung wird durch die folgenden Beispiele näher veranschaulicht.

Es werden die folgenden Abkürzungen verwendet:
- DMF: = N,N-Dimethylformamid
- DMAc: = N,N-Dimethylacetamid
- DMPU: = N,N'-Dimethylpropylenharnstoff
- DMI: = N,N'-Dimethylimidazolin-2-on
- THF: = Tetrahydrofuran
- NMP: = N-Methylpyrrolidon
- p-Me-C₆H₄: = 4-Toluyl
- p-MeO-C₆H₄: = 4-Methoxyphenyl
- p-Cl-C₆H₄: = 4-Chlorphenyl
- p-F-C₆H₄: = 4-Fluorphenyl
- Cy: = Cyclohexyl
- Ph = C₆H₅: = Phenyl
- JohnPhos: = 2-(Di-tert-butylphosphino)biphenyl
- Phen: = 1,10-Phenanthrolin
- DiPhPhen: = 4,7-Diphenyl-1,10-phenanthrolin

### Beispiele

### Beispiel 1: Synthese von 1-α-Noninsäure

In einem Kolben wurden (4,7-Diphenyl-1,10-phenanthrolin)bis(triphenylphosphin)-Kupfer(I) nitrat (19,7 mg, 0,02 mmol) und Cäsiumcarbonat (782 mg, 2,00 mmol) vorgelegt. Das Reaktionsgefäß wurde mit Stickstoff gespült und mit einer Septumkappe verschlossen. Anschließend wurde entgastes DMF (3,00 mL) zugesetzt, das entstandene Gemisch wurde bei Raumtemperatur für 5 min gerührt. Nach mehrmaligem Evakuieren und Rückfüllen des Reaktionsgefäßes mit CO₂ wurde 1-Octin (149 µL, 1,00 mmol) injiziert. Das Reaktionsgemisch wurde für 12 h bei 50 °C und 1 bar CO₂ Druck gerührt. Nach Ablauf der Reaktionszeit wurde das Reaktionsgemisch auf Raumtemperatur abgekühlt, mit Wasser verdünnt und dreimal mit je 100 ml *n*-Hexan extrahiert. Die wässrige Fraktion wurde mit verdünnter HCl (1 N, 10,0 mL) versetzt und anschließend dreimal mit je 100 mL Ethylacetat extrahiert. Die kombinierten organischen Fraktionen wurden mit LiCl Lösung (1 N, 10.0 mL) und gesättigter NaCl Lösung gewaschen, über Magnesiumsulfat getrocknet und filtriert. Das Lösungsmittel wurde am Rotationsverdampfer entfernt und ein farbloses Öl (146 mg, 95%) mit einem Siedepunkt von 123 °C / 3 mbar erhalten, das als das erwartete Reaktionsprodukt identifiziert wurde. ¹H NMR (600 MHz, CDCl₃) δ = 2,34 (t, *J*=7,2 Hz, 2 H), 1,55 - 1,60 (m, 2 H), 1,38 (d, *J*=7,6 Hz, 2 H), 1,25 - 1,32 (m, 4 H) 0,88 (t, *J=*7,0 Hz, 3 H) ppm. ¹³C NMR (151 MHz, CDCl₃) δ = 158,3, 92,8, 72,6, 31,1, 28,5, 27,3, 22,4, 18,8, 14,0 ppm. Anal. ber. für C₉H₁₄O₂: C, 70,1; H: 9.15; gefunden: C, 70.2; H, 9.3.

### Beispiel 2: Synthese von Phenylpropiolsäure

In einem Kolben wurden (4,7-Diphenyl-1,10-phenanthrolin)bis[tris(*p-*fluorphenyl)phosphin]-Kupfer(I) nitrat (10,9 mg, 0,01 mmol) und Cäsiumcarbonat (391 mg, 1,20 mmol) vorgelegt. Das Reaktionsgefäß wurde dann mit Stickstoff gespült und mit einer Septenkappe verschlossen. Anschließend wurde entgastes DMF (3,00 mL) zugesetzt, das entstandene Gemisch wurde bei Raumtemperatur für 5 min gerührt. Nach mehrmaligem evakuieren und begasen des Reaktionsgefäßes mit CO₂ wurde Phenylacetylen (110 µL, 1,00 mmol) injiziert. Das Reaktionsgemisch wurde für 12 h bei 35 °C und 5 bar CO₂ Druck in einem Stahlautoklaven gerührt. Nach Ablauf der Reaktionszeit wurde das Reaktionsgemisch auf Raumtemperatur abgekühlt, mit Wasser verdünnt und dreimal mit je 100 mL *n*-Hexan extrahiert. Die wässrige Fraktion wurde mit verdünnter HCl (1 N, 10,0 mL) versetzt wobei sich ein farbloser Feststoff bildete, welches nach Filtration, zur weiteren Aufreinigung aus Wasser und Ethanol umkristallisiert wurde. Der gereinigte farblose Feststoff (143 mg, 98%) mit einem Schmelzpunkt von 133-134 °C konnte als das gewünschte Reaktionsprodukt identifiziert werden. ¹H NMR (400 MHz, CDCL₃) δ = 7,04 (d, *J*=7,4 Hz, 2 H), 6,95 (t, *J*=7,2 Hz,1 H), 6,88 (t, *J*=7,2 Hz, 2 H) ppm. ¹³C NMR (101 MHz, Methanol-d4) δ = 156,7, 133,7, 131,6, 129,7 120,9, 86,4, 74,2 ppm. Anal. ber. für C₉H₆O₂: C, 73,9; H, 4,1; gefunden: C, 73,7; H, 4,3.

### Beispiele 3-22

Bei den Beispielen 3 - 22 wurden jeweils 1 mmol Alkin mit 1 mol% Cu(I)-Quelle (Formel 2) in Gegenwart von 1 mol% Ligand und 2,0 mmol Cs₂CO₃ umgesetzt. Dabei wurden jeweils 3 mL DMF verwendet. Die Produkte wurden nach Ablauf der Reaktionszeit mit Methyliodid verestert und mit GC/GC-MS charakterisiert. Die verwendeten Katalysatoren I bis X sind in Tabelle 1, die Ergebnisse sind in Tabelle 2 zusammengefasst.

**Tabelle 1: Kupfer(I)-komplexe**

| | | | |
|---|---|---|---|
| 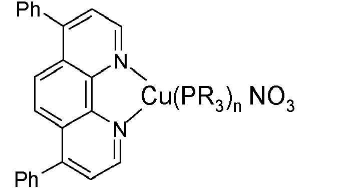 | | 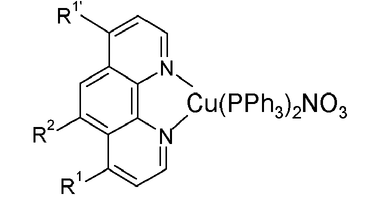 | |
| I: | R = C₆H₅; n= 2 | II: | R¹, R¹', R²= H |
| V: | R = p-MeO-C₆H₄; n= 2 | III: | R¹, R¹' = Cl; R²= H |
| VI: | R = p-Me-C₆H₄; n= 2 | IV: | R¹, R¹' = H; R²=NO₂ |
| VII: | R = Cy; n= 2 | | |
| VIII: | R = JohnPhos; n=1 | | |
| IX: | R = p-Cl-C₆H₄; n=2 | | |
| X: | R = p-F-C₆H₄; n= 2 | | |

**Tabelle 2:**

| | | | | | | | |
|---|---|---|---|---|---|---|---|
| | 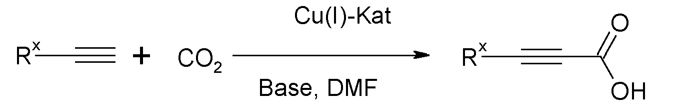 | | | | | | |
| | **1a:** | R^{x} = CH₃(CH₂)₅ | | | | | |
| | **1b:** | R^{X} = Phenyl | | | | | |

| # | 1 | Cu(I)-Kat | Base | CO₂ [bar] | T [°C] | t [h] | Ausbeute [%] |
|---|---|---|---|---|---|---|---|
| 3 | 1a | Cul/Phen | CS₂CO₃ | 1 | 100 | 8 | 52 |
| 4 | 1a | Cul/diPhPhen | CS₂CO₃ | 1 | 100 | 8 | 64 |
| 5* | 1a | Cul/PPh₃ | Cs₂CO₃ | 1 | 100 | 8 | 40 |
| 6 | 1a | I | Cs₂CO₃ | 1 | 100 | 8 | 74 |
| 7 | 1a | I | Cs₂CO₃ | 1 | 80 | 8 | 80 |
| 8 | 1a | I | Cs₂CO₃ | 1 | 50 | 8 | 92 |
| 9^{a),b)} | 1a | I | Cs₂CO₃ | 1 | 50 | 8 | 93 |
| 10^{b)} | 1b | I | Cs₂CO₃ | 1 | 50 | 8 | 65 |
| 11^{b)} | 1b | I | Cs₂CO₃ | 5 | 35 | 8 | 85 |
| 12^{b)} | 1b | I | Cs₂CO₃ | 5 | 35 | 2 | 52 |
| 13^{b)} | 1b | II | Cs₂CO₃ | 5 | 35 | 2 | 53 |
| 14^{b)} | 1b | III | Cs₂CO₃ | 5 | 35 | 2 | 43 |
| 15^{b)} | 1b | IV | Cs₂CO₃ | 5 | 35 | 2 | 43 |
| 16^{b)} | 1b | V | Cs₂CO₃ | 5 | 35 | 2 | 49 |
| 17^{b)} | 1b | VI | Cs₂CO₃ | 5 | 35 | 2 | 52 |
| 18^{b)} | 1b | VII | Cs₂CO₃ | 5 | 35 | 2 | 46 |
| 19^{b)} | 1b | VIII | Cs₂CO₃ | 5 | 35 | 2 | 22 |
| 20^{b)} | 1b | IX | Cs₂CO₃ | 5 | 35 | 2 | 58 |
| 21^{b)} | 1b | X | Cs₂CO₃ | 5 | 35 | 2 | 85 |
| 22^{b)} | 1b | X | Cs₂CO₃ | 5 | 35 | 8 | 99 |

| | | | | | | | |
|---|---|---|---|---|---|---|---|
| a) 2 mol% Cu(I)-Katalysator. b) 1,2 mmol Base. * nicht erfindungsgemäß | | | | | | | |

In den Beispielen 3 und 4 wurden die Katalysatoren *in situ* aus Kupfer(I)iodid und Stickstoffliganden erzeugt. Auch in diesen Fällen wird Produkt erhalten, die Ausbeuten bleiben jedoch hinter der des Beispiels 1 zurück, in dem für die gleiche Umsetzung ein präformierter Komplex mit Stickstoff- und Phosphinliganden eingesetzt wurde.

In Beispiel 5 wurden ausschließlich Phosphinliganden zugesetzt, und auch in diesem Fall sind die Ausbeuten deutlich geringer als in Beispiel 1.

In den Beispielen 6-9 wurden Temperaturen, Katalysatorbeladungen und Basenmenge variiert, es wird gezeigt, dass eine stöchiometrische Menge an Base ausreicht, und dass die Reaktion zwischen 50°C und 80°C besonders gute Ausbeuten liefert.

In den Beispielen 10-12 wurde gezeigt, dass für Alkine mit Arylsubstituenten bei erhöhtem CO₂ Druck um die 5 bar und bei geringeren Temperaturen um die 35 °C die besten Ausbeuten erzielt werden.

In den Beispielen 12-15 wurde demonstriert, dass Phenanthroline vorteilhafte Liganden sind, und dass Substituenten am Phenanthrolin einen positiven Einfluss auf die Ausbeuten haben können. Die besten Ausbeuten werden mit 4,7-Diphenyl-1,10-phenanthrolin erhalten.

In den Beispielen 4, 12, 16-21 wurde gezeigt, dass Phosphine als zusätzliche Liganden einen positiven Einfluss auf die Ausbeuten haben. Triarylphosphine sind vorteilhaft, die besten Ausbeuten wurden mit tri(*p*-Fluorphenyl)phosphin erhalten.

Im Beispiel 22 wurde demonstriert, dass das Gleichgewicht bei einer Reaktionszeit von 8 h fast quantitativ auf die Seite der Produkte verschoben wird, wenn ein (4,7-Dipheny-1,10-Iphenanthrolin)bis[tris(*p*-fluorphenyl)phosphin]-Kupfer(I) Komplex eingesetzt wird.

### Beispiele 23-27

Bei den Beispielen 23 - 27 (Tabelle 3) wurden jeweils 1,00 mmol Alkin mit 1 mol% CuI in Gegenwart von 1 mol% 4,7-Diphenyl-1,10-phenanthrolin und 6,00 mmol K₂CO₃ bei 60 °C für 2 h bei 10 bar CO₂ Druck umgesetzt. Dabei wurden jeweils 3,00 mL Lösungsmittel verwendet. Die Produkte wurden nach Ablauf der Reaktionszeit mit Methyliodid verestert und mit GC/GC-MS charakterisiert.

**Tabelle 3: Lösungsmitteleinfluss.**

| | | |
|---|---|---|
| 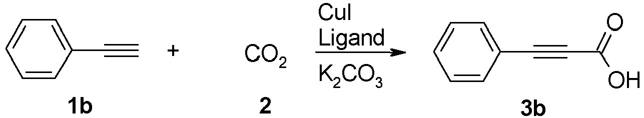 | | |

| # | Lösemittel | Ausbeute [%] |
|---|---|---|
| 23 | DMF | 88 |
| 24 | DMAc | 49 |
| 25 | DMPU | 16 |
| 26 | NMP | 49 |
| 27 | DMI | 30 |

Vergleichsbeispiel 28: Versuchte Herstellung von 1-α-Noninsäure unter Verwendung der von Inuoe beschriebenen Kupferkatalysatoren.

In einem Kolben wurden Kaliumcarbonat (830 mg, 6 mmol) und Kupfer(I)iodid(7,6 mg, 0,04 mmol) vorgelegt. Das Reaktionsgefäß wurde dann mit Stickstoff gespült und mit einer Septumkappe verschlossen. Anschließend wurde entgastes DMAc (3,00 mL) zugesetzt, das entstandene Gemisch wurde bei Raumtemperatur für 5 min gerührt. Nach mehrmaligem evakuieren und begasen des Reaktionsgefäßes mit CO₂ wurde 1-Octin (149 µL, 1,00 mmol) injiziert. Das Reaktionsgemisch wurde für 4h bei 100 °C und 1 bar CO₂ Druck gerührt. Nach Ablauf der Reaktionszeit wurde das Reaktionsgemisch abgekühlt, mit Methyliodid versetzt, und mittels Gaschromatographie untersucht. Trotz dieser Unterbrechung des Reaktionsgemisches vor der Aufarbeitung wurden neben 1-Octin lediglich 34% 1-α-Noninäure in Form des korrespondierenden Methylesters nachgewiesen. Dies bestätigt, dass eine Darstellung von Propiolsäuren nach dem Verfahren von Inuoe aufgrund der ungünstigen Gleichgewichtslage und den daraus resultierenden unbefriedigenden Ausbeuten wenig effizient wäre.

Bei Verwendung von 1,2 mmol Base und 0,01 mmol Kupfer(I)iodid wurde unter ansonsten identischen Bedingungen neben 1-Octin lediglich 18% 1-α-Noninsäure in Form des korrespondierenden Methylesters nachgewiesen. Der Vergleich mit Beispiel 1, in dem das Produkt bei gleich großen Mengen an Base und Katalysator in 95% Ausbeute erhalten wurde, verdeutlicht den Fortschritt, der durch das neue Katalysatorsystem und die neuen Reaktionsbedingungen erzielt wurde.

### Beispiele 29-32

Allgemeine Versuchsbeschreibung für die Synthese aliphatischer Propiolsäuren:
In einem Kolben werden (4,7-Diphenylphenanthrolin)bis(triphenylphosphin)-Kupfer(I) nitrat (19,7 mg, 0,02 mmol) und Cäsiumcarbonat (782 mg, 2,00 mmol) vorgelegt. Das Reaktionsgefäß wird dann mit Stickstoff gespült und mit einer Septumkappe verschlossen. Anschließend wird entgastes DMF (3,00 mL) zugesetzt und das entstandene Gemisch wird bei Raumtemperatur für 5 min gerührt. Nach mehrmaligem Evakuieren und Rückfüllen des Reaktionsgefäßes mit CO₂ wird das aliphatische Alkin (1,00 mmol) injiziert. Das Reaktionsgemsich wird anschließend für 12 h bei 50 °C und 1 bar CO₂ Druck gerührt. Nach Ablauf der Reaktionszeit wird das Reaktionsgemisch auf Raumtemperatur abgekühlt.

### Aufarbeitung:

Das Reaktionsgemisch wird mit Wasser verdünnt und dreimal mit je 100 ml *n*-Hexan extrahiert. Die wässrige Fraktion wird mit verdünnter HCl (1 N, 10,0 mL) versetzt und anschließend dreimal mit je 100 mL Ethylacetat extrahiert. Die kombinierten organischen Fraktionen werden mit LiCl Lösung (1 N, 10,0 mL) und gesättigter NaCl Lösung gewaschen, über Magnesiumsulfat getrocknet und filtriert. Das Lösungsmittel wird am Rotationsverdampfer entfernt, das Produkt bleibt als Feststoff oder Öl zurück.

### Beispiel 29: 4-Cyclohexylbut-2-insäure

4-Cyclohexylbut-2-insäure wurde nach der allgemeinen Versuchsbeschreibung aus 3-Cyclohexyl-1-propin (122 mg, 1,00 mmol) hergestellt. Nach Umkristallisation aus Wasser und Ethanol erhält man 4-Cyclohexylbut-2-insäure als farbloser Feststoff (141 mg, 85%) mit einem Schmelzpunkt von 85 °C. ¹H NMR (400 MHz, CDCl₃): δ = 10,46 (s, 1 H), 2,24 (d, J=6,7 Hz, 2 H), 1,79 (d, J=12,9 Hz, 2 H), 1,71 (d, J=12,9 Hz, 2 H), 1,64 (d, J=12,1 Hz, 1 H), 1,56 (ddd, J=10,7, 7,1, 4,1 Hz, 1 H), 1,17 - 1,26 (m, 2 H), 0,95 - 1,06 (m, 2 H) ppm. ¹³C NMR (101 MHz, CDCl₃): δ = 158,4, 91,8, 80,6, 77,4, 77,1, 76,8, 36,6, 32,7, 26,5, 26,0, 26,0, 21,1, 14,2 ppm. Anal. ber. für C₁₀H1₄O_{2:} C, 72,2; H, 8,5. Gefunden: C, 71,9; H, 8,1.

### Beispiel 30: 4-Methoxy-2-butinsäure

4-Methoxy-2-butinsäure wurde nach der allgemeinen Versuchsbeschreibung aus 3-Methoxy-1-propin (84 µL, 1,00 mmol) hergestellt. Man erhält 4-Methoxy-2-butinsäure (66 mg, 58%) als farbloses Öl. Die analytischen Daten (NMR, IR) stimmten mit den in der Literatur angegebenen Werten für 4-Methoxy-2-butinsäure [CAS: 24303-68-8] überein.

### Beispiel 31: 4-Methylpent-4-en-2-insäure

4-Methylpent-4-en-2-insäure wurde nach der allgemeinen Versuchsbeschreibung aus 2-Methyl-1-buten-3-in (98 µL, 1,00 mmol) hergestellt. Man erhält 4-Methylpent-4-en-2-insäure (106 mg, 97%) als farbloses Öl. Die analytischen Daten (NMR, IR) stimmten mit den in der Literatur angegebenen Werten für 4-Methylpent-4-en-2-insäure [CAS: 5963-81-5] überein.

### Beispiel 32: 5-Phenylpent-2-insäure

5-Phenylpent-2-insäure wurde nach der allgemeinen Versuchsbeschreibung aus 4-Phenyl-1-butin (141 µL, 1,00 mmol) hergestellt. Man erhält 5-Phenylpent-2-insäure (170 mg, 97%) als farbloses Öl. Die analytischen Daten (NMR, IR) stimmten mit den in der Literatur angegebenen Werten für 5-Phenylpent-2-insäure [CAS: 3350-93-4] überein.

### Beispiele 33-40

Für aromatische Propiolsäuren wurden bessere Ausbeuten erhalten, wenn etwas höhere CO₂ Drücke verwendet wurden. Diese Druckerhöhung hat bei der Herstellung aliphatischer Propiolsäuren keine Vorteile.

Allgemeine Versuchsbeschreibung für die Synthese aromatischer Propiolsäuren:
In einem Kolben werden (4,7-Diphenylphenanthrolin)bis[tris(p-fluorphenyl)phosphin]-Kupfer(I) nitrat (10,9 mg, 0,01 mmol) und Cäsiumcarbonat (391 mg, 1,20 mmol) vorgelegt. Das Reaktionsgefäß wird dann mit Stickstoff gespült und mit einer Septumkappe verschlossen. Anschließend wird entgastes DMF (3,00 mL) zugesetzt, das entstandene Gemisch wird bei Raumtemperatur für 5 min gerührt. Nach mehrmaligem Evakuieren und Rückfüllen des Reaktionsgefäßes mit CO₂ wird das aromatische Alkin (1,00 mmol) injiziert. Das Reaktionsgemisch wird anschließend für 12 h bei 35 °C und 5 bar CO₂ Druck in einem Stahlautoklaven gerührt. Nach Ablauf der Reaktionszeit wird das Reaktionsgemisch auf Raumtemperatur abgekühlt.

### Aufarbeitung:

Die Reaktionslösung wird mit Wasser verdünnt und dreimal mit je 100 ml *n*-Hexan extrahiert. Die Wässrige Fraktion wird mit verdünnter HCl (1 N, 10,0 mL) versetzt wobei sich ein Feststoff bildet, welches nach Filtration, zur weiteren Aufreinigung aus Wasser und Ethanol umkristallisiert wird.

### Beispiel 33: (4-Methylphenyl)-propiolsäure

(4-Methylphenyl)propiolsäure wurde nach der allgemeinen Versuchsbeschreibung aus (4-Methylphenyl)acetylen (127 µL, 1,00 mmol) hergestellt. Man erhält (4-Methylphenyl)propiolsäure (160 mg, 99%) als farbloser Feststoff. Die analytischen Daten (NMR, IR) stimmten mit den in der Literatur angegebenen Werten für (4-Methylphenyl)propiolsäure [CAS: 2227-58-9] überein.

### Beispiel 34: (4-Methoxyphenyl)propiolsäure

(4-Methoxyphenyl)propiolsäure wurde nach der allgemeinen Versuchsbeschreibung aus (4-Methoxyphenyl)acetylen (134 µL, 1,00 mmol) hergestellt. Man erhält (4-Methoxyphenyl)propiolsäure (143 mg, 81%) als farbloser Feststoff. Die analytischen Daten (NMR, IR) stimmten mit den in der Literatur angegebenen Werten für (4-Methoxyphenyl)propiolsäure [CAS: 2227-57-8] überein.

### Beispiel 35: (4-Trifluormethylphenyl)propiolsäure

(4-Trifluormethylphenyl)propiolsäure wurde nach der allgemeinen Versuchsbeschreibung aus 4-Ethinyl-a,a,a-Trifluortoluol (168 µL, 1,00 mmol) hergestellt. Man erhält (4-Trifluormethylphenyl)propiolsäure (214 mg, 99%) als farbloser Feststoff. Die analytischen Daten (NMR, IR) stimmten mit den in der Literatur angegebenen Werten für (4-Trifluormethylphenyl)propiolsäure [CAS: 3792-88-9] überein.

### Beispiel 36: (3-Brom-4-methoxyphenyl)propiolsäure

(3-Brom-4-methoxyphenyl)propiolsäure wurde nach der allgemeinen Versuchsbeschreibung aus 2-Brom-4-ethinylanisol (218 µL, 1,00 mmol) hergestellt. Man erhält (3-Brom-4-methoxyphenyl)propiolsäure (159 mg, 62%) als farbloser Feststoff mit einem Schmelzpunkt von 50 °C. ¹H NMR (600 M Hz, Ethanol-d₆): δ = 6,60 (s, 1 H), 6,45 (s, 1 H), 5,98 (s, 1 H), 2,81 (s, 3 H) ppm. ¹³C NMR (151 MHz, Ethanol-d₆): δ = 158,1,137,1, 136,1, 133,9, 132,4, 112,0, 111,7, 111,3, 81,4, 77,4 ppm. Anal. ber. für C₁₀H₇BrO₃: C, 47,1; H, 2,7. Gefunden: C, 4,4; H, 2,9.

### Beispiel 37: (3-Chlorphenyl)propiolsäure

(3-Chlorphenyl)propiolsäure wurde nach der allgemeinen Versuchsbeschreibung aus 3-Chlor-1-ethinylbenzol (127 µL, 1,00 mmol) hergestellt. Man erhält (3-Chlorphenyl)propiolsäure (155 mg, 86%) als farbloser Feststoff. Die analytischen Daten (NMR, IR) stimmten mit den in der Literatur angegebenen Werten für (3-Chlorphenyl)propiolsäure [CAS: 7396-28-3] überein.

### Beispiel 38: (2-Methylphenyl)propiolsäure

(2-Methylphenyl)propiolsäure wurde nach der allgemeinen Versuchsbeschreibung aus (2-Methylphenyl)acetylen (127 µL, 1,00 mmol) hergestellt. Man erhält (2-Methylphenyl)propiolsäure (139 mg, 87%) als farbloser Feststoff. Die analytischen Daten (NMR, IR) stimmten mit den in der Literatur angegebenen Werten für (2-Methylphenyl)propiolsäure [CAS: 7515-27-7] überein.

### Beispiel 39: (2-Methoxyphenyl)propiolsäure

(2-Methoxyphenyl)propiolsäure wurde nach der allgemeinen Versuchsbeschreibung aus (2-Methoxyphenyl)acetylen (129 µL, 1,00 mmol) hergestellt. Man erhält (2-Methoxyphenyl)propiolsäure (130 mg, 74%) als farbloser Feststoff. Die analytischen Daten (NMR, IR) stimmten mit den in der Literatur angegebenen Werten für (2-Methoxyphenyl)propiolsäure [CAS: 7342-00-9] überein.

### Beispiel 40: (4-Propylphenyl)propiolsäure

(4-Propylphenyl)propiolsäure wurde nach der allgemeinen Versuchsbeschreibung aus (4-Propylphenyl)acetylen (158 µL, 1,00 mmol) hergestellt. Man erhält (4-Propylphenyl)propiolsäure (140 mg, 74%) als farbloser Feststoff mit einem Schmelzpunkt von 155-156 °C. ¹H NMR (400 M Hz, Methanol-d₄): δ = 7,38 (d, J=7,8 Hz, 2 H), 7,14, (d, J=7,8 Hz, 2 H), 2,52 (t, J=7,6 Hz, 2 H), 1,49 - 1,59 (m, 2 H), 0,83 (t, *J*=7,2 Hz, 3 H) ppm. ¹³C NMR (101 MHz, Methanol-d₄): δ = 156,6, 147,2, 133,7, 129,8, 117,9, 86,8, 81,3, 38,8, 25,2, 13,8 ppm. Anal. ber. für C₁₂H₁₂O₂: C, 76,5; H, 6,4. Gefunden: C, 76,3; H, 6,7.

### Beispiel 41: Carboxylierung von Acetylen

In einem Kolben wurden (4,7-Diphenylphenanthrolin)bis[tri-phenylphosphin]-Kupfer(I) nitrat (21,3 mg, 0,02 mmol), 1-Bromhexan (282 µL, 2,00 mmol) und Cäsiumcarbonat (782 mg, 2,40 mmol) vorgelegt. Das Reaktionsgefäß wurde dann mit Stickstoff gespült und mit einer Septumkappe verschlossen. Anschließend wurde entgastes DMF (3,00 mL) zugesetzt, das entstandene Gemisch wird bei Raumtemperatur für 5 min gerührt und mehrmals mit CO₂ begast und evakuiert. Das Reaktionsgefäß wurde in ein Stahlautoklav gegeben und es wurde ein Acetylendruck von 1 bar eingestellt. Das Reaktionsgemisch wurde anschließend bei 60 °C und 5 bar CO₂ Druck für 2 h gerührt. Nach Ablauf der Reaktionszeit wurde das Reaktionsgemisch auf Raumtemperatur abgekühlt, 50 µL *n*-Tetradecan hinzugegeben, eine 0.25 mL Probe entnommen, in 3 mL Ethylessigester und 2 mL Wasser gewaschen, 0.25 mL entnommen, durch eine Pipette mit MgSO₄ gefiltert und analysiert. Es konnten 7,5 mg Acetylencarbonsäure und 10,4 mg Acetylendicarbonsäure in Form der n-Hexylester im Reaktionsgemisch nachgewiesen werden.

### Beispiel 42: Carboxylierung von Acetylen mit anschließender Zugabe von 1-Bromhexan

In einem Kolben wurden (4,7-Diphenylphenanthrolin)bis[tri-phenylphosphin]-Kupfer(I) nitrat (21,3 mg, 0,02 mmol) und Cäsiumcarbonat (782 mg, 2,40 mmol) vorgelegt. Das Reaktionsgefäß wurde dann mit Stickstoff gespült und mit einer Septumkappe verschlossen. Anschließend wurde entgastes DMF (3,00 mL) zugesetzt, das entstandene Gemisch wird bei Raumtemperatur für 5 min gerührt und mehrmals mit CO₂ begast und evakuiert. Das Reaktionsgefäß wurde in ein Stahlautoklav gegeben und es wurde ein Acetylendruck von 1 bar eingestellt. Das Reaktionsgemisch wurde anschließend bei 60 °C und 5 bar CO₂ Druck für 2 h gerührt. Nach Ablauf der Reaktionszeit wurde 1-Bromhexan (282 µL, 2,00 mmol) zu dem Reaktionsgemisch zugegeben und eine weitere Stunde bei 60 °C erhitzt. Anschließend wurde auf Raumtemperatur abgekühlt, 50 µL *n*-Tetradecan hinzugegeben, eine 0.25 mL Probe entnommen, in 3 mL Ethylessigester und 2 mL Wasser gewaschen, 0.25 mL entnommen, durch eine Pipette mit MgSO₄ gefiltert und analysiert. Es konnten 7,3 mg Acetylencarbonsäure und 10,5 mg Acetylendicarbonsäure in Form der n-Hexylester im Reaktionsgemisch nachgewiesen werden.

### Beispiele 43-49

Allgemeine Vorschrift für die Herstellung von Kupfer-Phosphin-Komplexen:
In einem Schlenkgefäß werden 2,00 mL Ethanol vorgelegt und zum Rückfluss erhitzt. Anschließend wird unter N₂ Atmosphäre das Phosphin (3,00 mmol) langsam bis zum vollstängigem Lösen hinzugegeben. Zu diesem Gemisch wird dann Kupfer(II) nitrat trihydrat (242 mg, 1.00 mmol) über einen Zeitraum von 20 min portionsweise hinzugegeben. Nach vollständiger Zugabe wird das Reaktionsgemisch für 30 min zum Sieden erhitzt wobei ein Niederschlag entsteht. Der Niederschlag wird anschließend filtriert und mit Ethanol (2 x 10.0 mL) und kaltem (0 °C) Diethylether gewaschen. Anschließend wird im Vakuum (2 x 10⁻³ mmHg) getrocknet.

### Beispiel 43: Bis(triphenylphosphin)-kupfer(I)-nitrat.

Bis(triphenylphosphin)-kupfer(I)-nitrat wurde nach der allgemeinen Versuchsbeschreibung aus Triphenylphosphin (787 mg, 3.00 mmol) hergestellt. Man erhielt Bis(triphenylphosphin)-kupfer(I)-nitrat (480 mg, 74%) als hellgrüner Feststoff. ³¹P NMR (162 MHz, DMSO-d₆): δ= -3.56 (s, 2 P) ppm. Anal. ber. für C₃₆H₃₀CuNO₃P₂: C, 66.5; H, 4.6; N; 2.1. Gefunden: C, 66.1; H, 4.5; N, 2.1.

### Beispiel 44: Bis(tris(p-methoxyphenyl)phosphin)-kupfer(I)-nitrat

Bis(tris(*p*-methoxyphenyl)phosphin)-kupfer(I)-nitrat wurde nach der allgemeinen Versuchsbeschreibung aus Tris(*p*-methoxyphenyl)phosphin (1.05 g, 3.00 mmol) hergestellt. Man erhielt Bis(tris(*p*-methoxyphenyl)phosphin)-kupfer(I)-nitrat (712 mg, 86%) als hellgrüner Feststoff. ³¹P NMR (162 MHz, CDCl₃): δ= 28.69 (s, 1 P), 19.36 (s, 1 P) ppm. Anal. ber. für C₆₆H₅₈CuN₃O₉P₂: C, 68.2; H; 5.0; N, 3.6. Gefunden: C, 67.4; H, 5.3; N, 3.5.

### Beispiel 45: Bis(tri-p-tolylphosphin)-kupfer(I)-nitrat

Bis(tri-*p*-tolylphosphin)-kupfer(I)-nitrat wurde aus tri-*p*-tolylphospin (913 mg, 3.00 mmol) hergestell. Man erhält Bis(tri-*p*-tolylphosphin)-kupfer(I)-nitrat (390 mg, 54%) als hellgrüner Feststoff. ³¹P NMR (162 MHz, CDCl₃): δ 27.06 (s, 1 P) -36.45 (s, 1 P) ppm. Anal. ber. für C₄₂H₄₂CuNO₃P₂: C, 68.7; H, 5.7; N, 1.9. Gefunden: C, 68.4; H, 5.3; N, 1.9.

### Beispiel 46: Bis(tricyclohexylphosphin)-kupfer(I)-nitrat

Bis(tricyclohexylphosphin)-kupfer(I)-nitrat wurde aus tricyclohexylphosphin (841 mg, 3.00 mmol) hergestellt. Man erhält Bis(tricyclohexylphosphin)-kupfer(I)-nitrat (389 mg, 57%) als gelber Feststoff. ³¹P NMR (162 MHz, COCl₃): δ□ 13.22 (s, 2 P) ppm. Anal. ber. für C₃₆H₆₆CuNO₃P₂: C, 63.0; H, 9.7; N, 2.0. Gefunden: C, 62.8; H, 9.5; N, 2.1.

### Beispiel 47: [(O-biphenyl)di-tert-butylphosphin]Kupfer(I)-nitrat

[(*O*-biphenyl)di-*tert*-butylphosphin]Kupfer(I)-nitrat wurde aus (*O*-biphenyl)di-*tert-*butylphosphin (1.34 g, 3.00 mmol) hergestellt. Man erhält [(*O*-biphenyl)di-*tert-*butylphosphin]Kupfer(I)-nitrat (630 mg, 99%) als farbloser Feststoff. ³¹P NMR (162 MHz, CDCl₃): δ= 51.54 (s, 2 P) ppm. Anal. Calcd. for C₄₀H₅₄CuNO₃P: C, 56.7; H, 6.4; N, 3.3. Found: C, 57.0; H, 6.3; N, 3.4.

### Beispiel 48: Bis[tris(p-chlorphenyl)phosphin]-kupfer(I)-nitrat

Bis[tris(*p*-chlorphenyl)phosphin]-kupfer(I)-nitrat wurde aus Tris(*p*-chlorphenyl)phosphin (1.10 g, 3.00 mmol) hergestellt. Man erhält Bis[tris(*p*-chlorphenyl)phosphin]-kupfer(I)-nitrat (247 mg, 39%) als farbloser Feststoff. Anal. ber. für C₃₆H₂₄C₁₆CuNO₃P₂: C, 50.47; H, 2.8; N, 1.6. Gefunden: C, 50.19; H, 3.0; N, 2.0.

### Beispiel 49: Bis[tris(p-fluorphenyl)phosphin]-kupfer(I)-nitrat

Bis[tris(*p*-fluorphenyl)phosphin]-kupfer(I)-nitrat wurde aus Tris(*p*-fluorophenyl)phosphin (949 mg, 3.00 mmol) hergestellt. Man erhält Bis[tris(*p*-fluorphenyl)phosphin]-kupfer(I)-nitrat (560 mg, 74%) als farbloser Feststoff. ³¹P NMR (162 MHz, CDCl₃): δ= 19.86 (s, 2 P) ppm. Anal. ber. für C₃₆H₂₄CuF₆NO₃P₂: C, 57.0; H, 3.2; N, 1.8. Gefunden: C, 57.3; H, 3.2; N, 2.2.

### Beispiele 50-58: Allgemeine Versuchsbeschreibung für die Synthese von Kupfermischligand-komplexe

In einem Schlenkgefäß wird der Kupfer-Phosphin Komplex (1,00 mmol) in 10,0 mL CHCl₃ vorgelegt. Zu dieser Lösung wird das Phosphin (1,00 mmol) bis zum vollständigen Lösen hinzugefügt. Danach wird eine Lösung des N-Liganden (1,00 mmol) in 2 mL CHCl₃ über einen Zeitraum von 30 min hinzugegeben. Anschließend wird weitere 30 min bei Raumtemperatur gerührt. Nach dem Entfernen des CHCl₃ *in vacuo* wird der erhaltene Feststoff aus CH₂Cl₂ und Et₂O umkristallisiert.

### Beispiel 50: (4,7-Diphenyl-1,10-phenanthrolin)bis(triphenylphosphin)-kupfer(I)-nitrat

(4,7-Diphenyl-1,10-phenanthrolin)bis(triphenylphosphin)-kupfer(I)-nitrat wurde aus Bis(triphenylphosphin)-kupfer(I)-nitrat (650 mg, 1,00 mmol), 4,7-Diphenyl-1,10-phenanthrolin (339 mg, 1,00 mmol) und Triphenylphosphin (262 mg, 1,00 mmol) hergestellt. Man erhält (4,7-Diphenyl-1,10-phenanthrolin)bis(triphenylphosphin)-kupfer(I)-nitrat (980 mg, 99%) als gelber Festoff. ³¹P NMR (162 MHz, CDCl₃) δ= 3.32 (s, 1 P) ppm. Anal. ber. für C₄₈H₃₆Cl₂CuN₃O₃P₂: C, 59.8, H, 3.9, N, 4.3. Gefunden: C, 59.0, H, 3.8, N, 4.6.

Beispiel 51: (4,7-Dichlor-1,10-phenanthrolin)bis(triphenylphosphin)-kupfer(I)-nitrat (4,7-Dichlor-1,10-phenanthrolin)bis(triphenylphosphin)-kupfer(I)-nitratwurde aus Bis(triphenylphosphin)-kupfer(I)-nitrat (650 mg, 1,00 mmol), 4,7-Dichlor-1,10-phenanthrolin (249 mg, 1,00 mmol) und Triphenylphosphin (262 mg, 1,00 mmol) hergestellt. Man erhält (4,7-Dichlor-1,10-phenanthrolin)bis(triphenylphosphin)-kupfer(I)-nitrat (980 mg, 99%) als hellbrauner Feststoff. ³¹P NMR (162 MHz, CDCl₃): δ= 2.62 (s, 2 P) ppm. Anal. ber. für C₄₈H₃₆Cₗ₂CuN₃O₃P₂: C, 59.8; H, 3.89; N, 4.3. Gefunden: C, 58.9; H, 3.8; N, 4.6.

Beispiel 52: (5-Nitro-1,10-phenanthrolin)bis(triphenylphosphin)-kupfer(I)-nitrat (5-Nitro-1,10-phenanthrolin)bis(triphenylphosphin)-kupfer(I)-nitrat wurde aus Bis(triphenylphosphin)-kupfer(I)-nitrat (650 mg, 1,00 mmol), 5-Nitro-1,10-phenanthrolin (232 mg, 1.00 mmol) und Triphenylphosphin (262 mg, 1,00 mmol) hergestellt. Man erhält (5-Nitro-1,10-phenanthrolin)bis(triphenylphosphin)-kupfer(I)-nitrat (723 mg, 83%) als orangefarbener Feststoff. ³¹P NMR (162 M Hz, DMSO-d₆): δ= -3.56 (s, 2 P) ppm. Anal. ber. für C₄₈H₃₇CuN₄O₅P₂: C, 65.8; H, 4.2; N, 6.4. Gefunden: C, 64.6; H, 4.2; N, 6.3.

### Beispiel 53: (4,7-Diphenyl-1,10-phenanthrolin)bis[tris(p-methoxyphenyl)phosphin]-kupfer(I)-nitrat

(4,7-Diphenyl-1,10-phenanthrolin)bis[tris(p-methoxyphenyl)phosphin]-kupfer(I)-nitrat wurde aus Bis(tris(*p*-methoxyphenyl)phosphin)-kupfer(I)-nitrat (830 mg, 1,00 mmol), 4,7-Diphenyl-1,10-phenanthrolin (339 mg, 1,00 mmol) und tris(*p-*methoxyphenyl)phosphin (352 mg, 1,00 mmol) hergestellt. Man erhält (4,7-Diphenyl-1,10-phenanthrolin)bis[tris(*p*-methoxyphenyl)phosphin]-kupfer(I)-nitrat als hellbrauner Feststoff (1,03 g, 89%). ³¹P N M R (162 M Hz, CDCl₃): δ= 28.69 (s, 1 P), 19.36 (s, 1 P) ppm. Anal. ber. für C₆₆H₅₈CuN₃O₉P₂: C, 68.1; H, 5.0; N, 3.4. Gefunden: C, 67.4; H, 5.3; N, 3.4.

Beispiel 54: (4,7-Diphenyl-1,10-phenanthrolin)bis(tri-*p*-tolylphosphin)-kupfer(I)-nitrat (4,7-Diphenyl-1,10-phenanthrolin)bis(tri-*p*-tolylphosphin)-kupfer(I)-nitrat wurde aus Bis(tri-*p*-tolylphosphin)-kupfer(1)-nitrat (734 mg, 1,00 mmol), 4,7-Diphenyl-1,10-phenanthrolin (339 mg, 1,00 mmol) und Tri-*p*-tolylphosphine (304 mg, 1,00 mmol) hergestellt. Man erhält (4,7-Diphenyl-1,10-phenanthrolin)bis(tri-*p*-tolylphosphin)-kupfer(I)-nitrat (891 mg, 76%) als hellbrauer Feststoff. ³¹P NMR (162 MHz, CDCl₃): δ= 20.61 (s, 2 P) ppm. Anal. ber. für C₆₆H₅₈CuN₃O₃P₂: C, 69.9; H, 5.25; N, 3.6. Gefunden: C, 71.1; H, 5.4; N, 3.9.

Beispiel 55: (4,7-Diphenyl-1,10-phenanthrolin)bis(tricyclohexylphosphin)-kupfer(I)-nitrat (4,7-Diphenyl-1,10-phenanthrolin)bis(tricyclohexylphosphin)-kupfer(I)-nitrat wurde aus Bis(tricyclohexylphosphin)-kupfer(I)-nitrat (686 mg, 1,00 mmol), 4,7-Diphenyl-1,10-phenanthrolin (339 mg, 1,00 mmol) und Tricyclohexylphosphin (280 mg, 1,00 mmol) hergestellt. Man erhält (4,7-Diphenyl-1,10-phenanthrolin)bis(tricyclohexylphosphin)-kupfer(I)-nitrat (867 mg, 85%) als gelber Feststoff. ³¹P NMR (162 MHz, CDCl₃): δ= 50.11 (s, 1 P), 33.76 (s, 1 P) ppm. Anal. ber. für C₆₀H₈₂CuN₃O₃P₂: C, 70.7; H, 8.1; N, 4.1. Gefunden: C, 69.8; H, 8.1; N, 3.9.

### Beispiel 56: [(4,7-Diphenyl-1,10-phenanthrolin)-(o-biphenylyl)di-tert-butylphosphin]-kupfer(I)-nitrat

[(4,7-Diphenyl-1,10-phenanthrolin)-(*o*-biphenylyl)di-*tert*-butylphosphin]-kupfer(I)-nitrat wurde aus [(*O*-biphenylyl)di-*tert*-butylphosphin]-kupfer(I)-nitrat (423 mg, 1,00 mmol), 4,7-Diphenyl-1,10-phenanthrolin (339 mg, 1,00 mmol) und (*O*-biphenyl)di-*tert-*butylphosphin (298 mg, 1,00 mmol) hergestellt. Man erhält: [(4,7-Diphenyl-1,10-phenanthrolin)-(*o*-biphenylyl)di-*tert*-butylphosphin]-kupfer(I)-nitrat (748 mg, 99%) als gelber Feststoff. ³¹P NMR (162 MHz, CDCl₃): δ= 33.23 (s, 1 P), 18.14 (s, 1 P) ppm. Anal. ber. für C₆₄H₇₀CuN₃O₃P₂: C, 69.8; H, 5.7; N, 5.5. Gefunden: C, 68.4; H, 5.8; N, 5.2.

### Beispiel 57: (4,7-Diphenyl-1,10-phenanthrolin)bis[tris(p-chlorphenyl)phosphin)]-kupfer(I)-nitrat

(4,7-Diphenyl-1,10-phenanthrolin)bis[tris(*p*-chlorphenyl)phosphin)]-kupfer(I)-nitrat wurde aus Bis[tris(*p*-chlorphenyl)phosphin]-kupfer(l) nitrat (856 mg, 1,00 mmol), 4,7-Diphenyl-1,10-phenanthrolin (339 mg, 1,00 mmol) und Tris(*p*-chlorphenyl)phosphin (366 mg, 1,00 mmol) hergestellt. Man erhält (4,7-Diphenyl-1,10-phenanthrolin)bis[tris(*p*-chlorphenyl)phosphin)]-kupfer(I)-nitrat als (852 mg, 72%) gelber Feststoff. ³¹P NMR (162 M Hz, CDCl₃): δ= -5.90 (s, 2 P) ppm. Anal. ber. für C₆₀H₄₀Cl₆CuN₃O₃P₂: C, 60.6; H, 3.4; N, 3.5. Gefunden: C, 60.3; H, 3.5; N, 3.9.

### Beispiel 58: (4,7-Diphenyl-1,10-phenanthrolin)bis[tris(p-fluorphenyl)phosphin]-kupfer(I)-nitrat

(4,7-Diphenyl-1,10-phenanthrolin)bis[tris(*p*-fluorphenyl)phosphin]-kupfer(I)-nitrat wurde aus Bis[tris(*p*-fluorphenyl)phosphin]-kupfer(I) nitrate (758 mg, 1,00 mmol), 4,7-Diphenyl-1,10-Phenanthrolin (339 mg, 1.00 mmol) und Tris(*p*-fluorphenyl)phosphin (316 mg, 1,00 mmol) hergestellt. Man erhält (4,7-Diphenyl-1,10-phenanthrolin)bis[tris(*p*-fluorphenyl)phosphin]-kupfer(I)-nitrat (1.4 g, 97%) als gelber Feststoff. ³¹P NMR (162 MHz, CDCl₃): δ= 19.84 (s, 2 P) ppm. Anal. ber. für C₆₀H₄₀CuF₆N₃O₃P₂: C, 66.1; H, 3.7; N, 3.8. Gefunden: C, 65.4; H, 3.8; N, 4.0.

## Patentansprüche

1. Verfahren zur Herstellung einer Propiolsäure oder eines Derivates davon durch Umsetzung eines terminalen Alkins mit Kohlendioxid, **dadurch gekennzeichnet, dass** die Umsetzung in Gegenwart einer Base und einem Kupferkomplex mit wenigstens einem mehrzähnigen Liganden durchgeführt wird, der wenigstens zwei zur gleichzeitigen Koordination mit Kupfer befähigte Atome oder Atomgruppen aufweist, die unter Stickstoff, Sauerstoff, Schwefel, Phosphor und Carben-Kohlenstoff ausgewählt sind und wobei der Kupferkomplex wenigstens einen weiteren Liganden aufweist, der unter Phosphinen ausgewählt ist.

2. Verfahren nach Anspruch 1, wobei als terminales Alkin eine Verbindung der Formel
R^{x}-C≡C-H
eingesetzt wird, worin,
R^{x} für Wasserstoff, COOR^{x1}, Alkyl, Alkenyl, Cycloalkyl, Heterocycloalkyl, Aryl, Hetaryl oder (R^{x2})₃Si steht und wobei Alkyl und Alkenyl unsubstituiert sind oder einen oder mehrere Substituenten, z. B. 1, 2, 3, 4 oder 5 Substituenten R^{x3} aufweisen und worin Cycloalkyl, Heterocycloalkyl, Aryl und Hetaryl unsubstituiert sind oder einen oder mehrere Substituenten, z. B. 1, 2, 3, 4 oder 5 Substituenten R^{x4} aufweisen, wobei
R^{x1} ausgewählt ist unter Wasserstoff, Alkyl, Cycloalkyl, Heterocycloalkyl, Aryl und Hetaryl, wobei die vier zuletzt genannten Reste unsubstituiert sind oder einen oder mehrere, z.B. 1, 2 oder 3, Reste aufweisen, die unter Hydroxy (= OH), Mercapto (= SH), NE¹E², C(O)NE¹E², Halogen, Nitro (= NO₂), Nitroso (= NO), Formyl (= C(=O)H), Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind,
R^{x2} ausgewählt ist unter Alkyl, Cycloalkyl, Heterocycloalkyl, Aryl und Hetaryl, wobei die vier zuletzt genannten Reste unsubstituiert sind oder einen oder mehrere, z.B. 1, 2 oder 3, Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind,
R^{x3} ausgewählt ist unter Halogen, Cyano, Hydroxy, Mercapto, Alkoxy, COOH, SO₃H, NE¹E², C(O)NE¹E², Acyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl und Hetaryloxycarbonyl, wobei die cyclischen Gruppen in den zwölf zuletzt genannten Resten unsubstituiert sind oder einen oder mehrere, z.B. 1, 2 oder 3, Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl, ausgewählt sind,
R^{x4} ausgewählt ist unter Halogen, Cyano, Nitro, Hydroxy, Merkapto, Alkoxy, COOH, SO₃H, NE¹E², C(O)NE¹E², Alkyl, Halogenalkyl, Acyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl und Hetaryloxycarbonyl, wobei die cyclischen Gruppen in den zwölf zuletzt genannten Resten unsubstituiert sind oder einen oder mehrere, z.B. 1, 2 oder 3, Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind,
worin E¹ und E² gleiche oder verschiedene Reste, ausgewählt unter Wasserstoff, Alkyl, Cycloalkyl, oder Aryl bedeuten oder E¹ und E² gemeinsam mit dem Stickstoffatom, an das sie gebunden sind, für gesättigtes Stickstoffheterocyclyl stehen, der unsubstistuiert ist oder einen oder mehrere Alkylgruppen als Substituenten aufweist.

3. Verfahren nach Anspruch 1, wobei Acetylen als terminales Alkin eingesetzt wird und Propiolsäure und/oder Acetylendicarbonsäure erhalten wird.

4. Verfahren nach einem der vorhergehenden Ansprüche, wobei der Ligand ein mehrzähniger Stickstoffligand ist, der wenigstens zwei zur Koordination mit Kupfer befähigte Stickstoffatome aufweist.

5. Verfahren nach Anspruch 4, wobei der mehrzähnige Stickstoffligand ein Gerüst der Formel I aufweist, 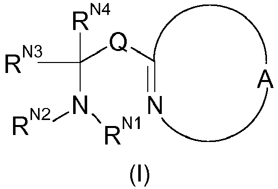 worin
A zusammen mit dem Fragment C=N, an das es gebunden ist, einen 5- bis 7-gliedrigen heterocyclischen Ring bildet, der mit einem, zwei oder drei weiteren Ringen anelliert sein kann;
Q für eine chemische Bindung oder eine verbrückende Gruppe mit einem, zwei oder drei Atomen steht; wobei die chemische Bindung oder verbrückende Gruppe teilweise oder vollständig Bestandteil eines oder mehrer Ringe sein kann, wobei im Falle einer cyclischen Gruppen Q, diese mit dem Ring A annelliert sein kann;
R^{N1} für Wasserstoff steht oder
R^{N1} mit R^{N4} eine chemische Bindung bildet,
R^{N2} für Alkyl, Cycloalkyl oder Aryl steht
R^{N3} für Wasserstoff, Alkyl, Cycloalkyl oder Aryl steht, wobei Alkyl unsubstituiert ist oder einen Rest aus der Gruppe Cycloalkyl oder Aryl trägt;
R^{N2} und R^{N3} gemeinsam mit den Atomen, an die sie gebunden sind, einen 5-bis 7-gliedrigen heterocyclischen Ring bilden, der mit einem, zwei oder drei weiteren Ringen anelliert sein kann;
R^{N4} für Wasserstoff steht oder abwesend ist oder mit R^{N1} eine chemische Bindung bildet.

6. Verfahren nach Anspruch 4, wobei der mehrzähnige Stickstoffligand die Formel IV aufweist, 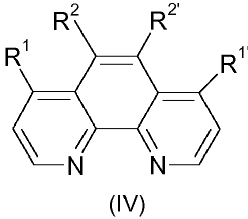
worin R¹ und R^{1'} unabhängig voneinander für Wasserstoff, Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Arylthio, Hetarylthio, Cycloalkoxycarbonyl, Heterocycloalkoxycarbonyl, Aryloxycarbonyl oder Hetaryloxycarbonyl stehen,
worin die cyclischen Gruppen in den vierzehn zuletzt genannten Resten unsubstituiert sind oder einen oder mehrere Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl, Hetaryloxycarbonyl ausgewählt sind,
worin E¹ und E² gleiche oder verschiedene Reste, ausgewählt unter Wasserstoff, Alkyl, Cycloalky und Aryl, bedeuten oder E¹ und E² gemeinsam mit dem Stickstoffatom, an das sie gebunden sind, für gesättigtes Stickstoffheterocyclyl stehen, der unsubstistuiert ist oder einen oder mehrere Alkylgruppen als Substituenten aufweist, und
R² und R^{2'} unabhängig voneinander für eine der für R¹ und R^{1'} angegebenen Bedeutungen aufweisen.

7. Verfahren nach Anspruch 6, wobei in der Formel IV beide Reste R¹ und R^{1'} für Phenyl stehen, das gegebenenfalls 1 oder 2 Reste trägt, die unter Halogen, Alkyl, Halogenalkyl, Alkoxy und Halogenalkoxy ausgewählt sind.

8. Verfahren nach Anspruch 7, wobei der Stickstoffligand 4,7-Diphenyl-1,10-phenanthrolin ist.

9. Verfahren nach Anspruch 1, wobei der wenigstens eine weitere Ligand unter Triarylphosphinen ausgewählt ist.

10. Verfahren nach Anspruch 9, wobei der wenigstens eine weitere Ligand tri(*p-*Fluorphenyl)phosphin ist.

11. Verfahren nach einem der vorhergehenden Ansprüche, wobei der Kupferkomplex als präformierter Kupferkomplex eingesetzt wird.

12. Verfahren nach einem der vorhergehenden Ansprüche, wobei eine Katalysatormenge von 0,001 mol% bis 20 mol%, gerechnet als Cu und bezogen auf das Alkin eingesetzt wird.

13. Verfahren nach einem der vorhergehenden Ansprüche, wobei die Umsetzung in Gegenwart eines Lösungsmittels, ausgewählt unter aliphatischen Kohlenwasserstoffen, aromatischen Kohlenwasserstoffen, Amiden, Harnstoffen, Nitrilen, Sulfoxiden, Sulfonen, Alkoholen, Estern, Carbonaten, Ethern und deren Gemischen, durchgeführt wird.

14. Verfahren nach einem der vorhergehenden Ansprüche, wobei die zur Base korrespondierende Säure bei 25°C in Wasser einen pKs-Wert aufweist, der wenigstens 3 pK-Einheiten unterhalb des pKs-Wertes des eingesetzten Alkins liegt.

15. Verfahren nach einem der vorhergehenden Ansprüche, wobei die Base unter Alkalihydroxiden, Erdalkalihydroxiden, Alkalicarbonaten, Erdalkalicarbonaten, Alkalibicarbonaten, Erdalkalibicarbonaten, Alkalioxiden, Erdalkalioxiden, Alkaliphosphaten, Erdalkaliphosphaten, Alkalihydrogenphosphaten, Erdalkalihydrogenphosphaten, Alkalifluoriden, Erdalkalifluoriden, Alkalicarboxylaten, Erdalkalicarboxylaten und Gemischen davon ausgewählt ist.

16. Verwendung von Kupfer(I)-Komplexen, umfassend einen mehrzähnigen Stickstoffliganden und wenigstens einen Phosphinliganden, wobei der mehrzähnigen Stickstoffligand die Formel IV' aufweist, 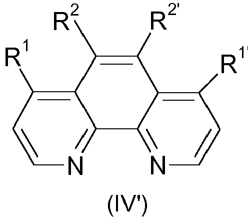
worin R¹ und R^{1'} unabhängig voneinander für Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Arylthio, Hetarylthio, Cycloalkoxycarbonyl, Heterocycloalkoxycarbonyl, Aryloxycarbonyl oder Hetaryloxycarbonyl stehen,
worin die cyclischen Gruppen in den vierzehn zuletzt genannten Resten unsubstituiert sind oder einen oder mehrere Reste aufweisen, die unter Hydroxy, Mercapto, NE¹E², C(O)NE¹E², Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl, Cycloalkyl, Heterocycloalkyl, Aryl, Hetaryl, Cycloalkoxy, Heterocycloalkoxy, Aryloxy, Hetaryloxy, Cycloalkoxycarbonyl, Heterocycloalkoxycarbonyl, Aryloxycarbonyl, Hetaryloxycarbonyl ausgewählt sind,
worin E¹ und E² gleiche oder verschiedene Reste, ausgewählt unter Wasserstoff, Alkyl, Cycloalky und Aryl, bedeuten oder E¹ und E² gemeinsam mit dem Stickstoffatom, an das sie gebunden sind, für gesättigtes Stickstoffheterocyclyl stehen, der unsubstistuiert ist oder einen oder mehrere Alkylgruppen als Substituenten aufweist, und
R² und R^{2'} unabhängig voneinander für eine der für R¹ und R^{1'} angegebenen Bedeutungen aufweisen oder für Wasserstoff stehen,
als Carboxylierungskatalysator für terminale Alkine.

17. Verwendung von Kupfer(I)-Komplexen nach Anspruch 16 der allgemeinen Formel
(N,N)CuLₙ⁺ X-
worin
(N,N) für einen zweizähnigen N,N-Liganden der Formel IV' steht,
L für einen Phosphinliganden steht,
n für eine ganze Zahl von 1 bis 3 steht, und
X⁻ für ein Äquivalent eines Anions steht.
als Carboxylierungskatalysator für terminale Alkine.

## Claims

1. A process for preparing a propiolic acid or a derivative thereof by reacting a terminal alkyne with carbon dioxide, wherein the reaction is carried out in the presence of a base and a copper complex which has at least one polydentate ligand which has at least two atoms or atom groups which are capable of coordinating simultaneously to copper and are selected from among nitrogen, oxygen, sulfur, phosphorus and carbene carbon, and the copper complex has at least one further ligand which is selected from among phosphines.

2. The process according to claim 1, wherein a compound of the formula
R^{x} -C≡C-H
where
R^{x} is hydrogen, COOR^{x1}, alkyl, alkenyl, cycloalkyl, heterocycloalkyl, aryl, hetaryl or (R^{x2})₃Si and alkyl and alkenyl are unsubstituted or have one or more substituents, e.g. 1, 2, 3, 4 or 5 substituents R^{x3} and cycloalkyl, heterocycloalkyl, aryl and hetaryl are unsubstituted or substituted by one or more substituents e.g. 1, 2, 3, 4 or 5 substituents, where
R^{x1} is selected from among hydrogen, alkyl, cycloalkyl, heterocycloalkyl, aryl and hetaryl, where the latter four radicals are unsubstituted or have one or more, e.g. 1, 2 or 3, radicals selected from among hydroxy (= OH), mercapto (= SH), NE¹E², C(O)NE¹E², halogen, nitro (= NO₂), nitroso (= NO), formyl (= C(=O)H), alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl and cycloalkyl,
R^{x2} is selected from among alkyl, cycloalkyl, heterocycloalkyl, aryl and hetaryl, where the latter four radicals are unsubstituted or have one or more, e.g. 1, 2 or 3, radicals selected from among hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl and cycloalkyl,
R^{x3} is selected from among halogen, cyano, hydroxy, mercapto, alkoxy, COOH, SO₃H, NE¹E^{c}, C(O)NE¹E², acyl, alkoxycarbonyl, cycloalkyl, heterocycloalkyl, aryl, hetaryl, cycloalkoxy, heterocycloalkoxy, aryloxy, hetaryloxy, cycloalkoxycarbonyl, heterocycloalkoxycarbonyl, aryloxycarbonyl and hetaryloxycarbonyl, where the cyclic groups in the latter twelve radicals are unsubstituted or have one or more, e.g. 1, 2 or 3, radicals selected from among hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl and cycloalkyl,
R^{x4} is selected from among halogen, cyano, nitro, hydroxy, mercapto, alkoxy, COOH, SO₃H, NE¹E², C(O)NE¹E², alkyl, haloalkyl, acyl, alkoxycarbonyl, cycloalkyl, heterocycloalkyl, aryl, hetaryl, cycloalkoxy, heterocycloalkoxy, aryloxy, hetaryloxy, cycloalkoxycarbonyl, heterocycloalkoxycarbonyl, aryloxycarbonyland hetaryloxycarbonyl, where the cyclic groups in the latter twelve radicals are unsubstituted or have one or more, e.g. 1, 2 or 3, radicals selected from among hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl and cycloalkyl,
where E¹ and E² are identical or different radicals selected from among hydrogen, alkyl, cycloalkyl and aryl or E¹ and E² together with the nitrogen atom to which they are bound form a saturated nitrogen heterocyclyl which is unsubstituted or has one or more alkyl groups as substituents,
is used as terminal alkyne.

3. The process according to claim 1, wherein acetylene is used as terminal alkyne and propiolic acid and/or acetylenedicarboxylic acid is/are obtained.

4. The process according to any of the preceding claims, wherein the ligand is a polydentate nitrogen ligand which has at least two nitrogen atoms which are capable of coordinating to copper.

5. The process according to claim 4, wherein the polydentate nitrogen ligand has a skeleton of the formula I, 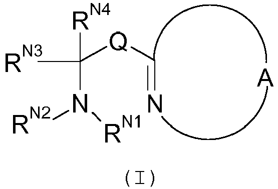 where
A together with the fragment C=N to which it is bound forms a 5- to 7-membered heterocyclic ring which may be fused with one, two or three further rings;
Q is a chemical bond or a bridging group having one, two or three atoms, where the chemical bond or bridging group can in part or in its entirety be a constituent of one or more rings, where in the case of a cyclic group Q this can be fused with the ring A;
R^{N1} is hydrogen or
R^{N1} together with R^{N4} forms a chemical bond,
R^{N2} is alkyl, cycloalkyl or aryl
R^{N3} is hydrogen, alkyl, cycloalkyl or aryl, where alkyl is unsubstituted or bears a radical selected from the group consisting of cycloalkyl or aryl;
R^{N2} and R^{N3} together with the atoms to which they are bound form a 5- to 7-membered heterocyclic ring which may be fused with one, two or three further rings;
R^{N4} is hydrogen or is absent or together with R^{N1} forms a chemical bond.

6. The process according to claim 4, wherein the polydentate nitrogen ligand has the formula IV, 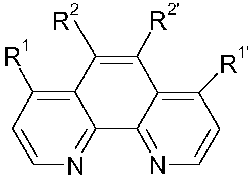 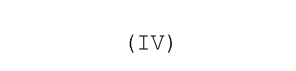
where R¹ and R^{1'} are each, independently of one another, hydrogen, hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl, cycloalkyl, heterocycloalkyl, aryl, hetaryl, cycloalkoxy, heterocycloalkoxy, aryloxy, hetaryloxy, arylthio, hetarylthio, cycloalkoxycarbonyl, heterocycloalkoxycarbonyl, aryloxycarbonyl or hetaryloxycarbonyl,
where the cyclic groups in the latter fourteen radicals are unsubstituted or have one or more radicals selected from among hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl, cycloalkyl, heterocycloalkyl, aryl, hetaryl, cycloalkoxy, heterocycloalkoxy, aryloxy, hetaryloxy, cycloalkoxycarbonyl, heterocycloalkoxycarbonyl, aryloxycarbonyl, hetaryloxycarbonyl,
where E¹ and E² are identical or different radicals selected from among hydrogen, alkyl, cycloalkyl and aryl or E¹ and E² together with the nitrogen atom to which they are bound form a saturated nitrogen heterocyclyl group which is unsubstituted or has one or more alkyl groups as substituents, and
R² and R^{2'} each have, independently of one another, one of the meanings indicated for R¹ and R^{1'}.

7. The process according to claim 6, wherein both the radicals R¹ and R^{1'} in the formula IV are phenyl which optionally bears 1 or 2 radicals selected from among halogen, alkyl, haloalkyl, alkoxy and haloalkoxy.

8. The process according to claim 7, wherein the nitrogen ligand is 4,7-diphenyl-1,10-phenanthroline.

9. The process according to claim 1, wherein the at least one further ligand is selected from among triarylphosphines.

10. The process according to claim 9, wherein the at least one further ligand is tri(*p*-fluorophenyl)phosphine.

11. The process according to any of the preceding claims, wherein the copper complex is used as preformed copper complex.

12. The process according to any of the preceding claims, wherein an amount of catalyst of from 0.001 mol% to 20 mol%, calculated as Cu and based on the alkyne, is used.

13. The process according to any of the preceding claims, wherein the reaction is carried out in the presence of a solvent selected from among aliphatic hydrocarbons, aromatic hydrocarbons, amides, ureas, nitriles, sulfoxides, sulfones, alcohols, esters, carbonates, ethers and mixtures thereof.

14. The process according to any of the preceding claims, wherein the acid corresponding to the base has a pKₐ in water at 25°C which is at least 3 pK units below the pKₐ of the alkyne used.

15. The process according to any of the preceding claims, wherein the base is selected from among alkali metal hydroxides, alkaline earth metal hydroxides, alkali metal carbonates, alkaline earth metal carbonates, alkali metal bicarbonates, alkaline earth metal bicarbonates, alkali metal oxides, alkaline earth metal oxides, alkali metal phosphates, alkaline earth metal phosphates, alkali metal hydrogenphosphates, alkaline earth metal hydrogenphosphates, alkali metal fluorides, alkaline earth metal fluorides, alkali metal carboxylates, alkaline earth metal carboxylates and mixtures thereof.

16. The use of copper(I) complexes comprising a polydentate nitrogen ligand and at least one phosphine ligand, where the polydentate nitrogen ligand has the formula IV', 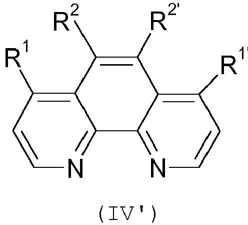
where R¹ and R^{1'} are each, independently of one another, hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl, cycloalkyl, heterocycloalkyl, aryl, hetaryl, cycloalkoxy, heterocycloalkoxy, aryloxy, hetaryloxy, arylthio, hetarylthio, cycloalkoxycarbonyl, heterocycloalkoxycarbonyl, aryloxycarbonyl or hetaryloxycarbonyl,
where the cyclic groups in the latter fourteen radicals are unsubstituted or have one or more radicals selected from among hydroxy, mercapto, NE¹E², C(O)NE¹E², halogen, nitro, nitroso, formyl, alkyl, haloalkyl, alkoxy, haloalkoxy, alkylthio, haloalkylthio, alkylcarbonyl, alkylcarbonyloxy, alkylcarbonylthio, haloalkylcarbonyl, alkoxycarbonyl, cycloalkyl, heterocycloalkyl, aryl, hetaryl, cycloalkoxy, heterocycloalkoxy, aryloxy, hetaryloxy, cycloalkoxycarbonyl, heterocycloalkoxycarbonyl, aryloxycarbonyl, hetaryloxycarbonyl,
where E¹ and E² are identical or different radicals selected from among hydrogen, alkyl, cycloalkyl and aryl or E¹ and E² together with the nitrogen atom to which they are bound form a saturated nitrogen heterocyclyl group which is unsubstituted or has one or more alkyl groups as substituents, and
R² and R^{2'} each have, independently of one another, one of the meanings indicated for R¹ and R^{1'} or are hydrogen, as carboxylation catalyst for terminal alkynes.

17. The use of copper(I) complexes according to claim 16 of the general formula
(N,N)CuLₙ⁺ X⁻
where
(N,N) represents a bidentate N,N-ligand of the formula IV',
L is a phosphine ligand,
n is an integer from 1 to 3, and
X⁻ is one equivalent of an anion,
as carboxylation catalyst for terminal alkynes.

## Revendications

1. Procédé pour la préparation d'un acide propiolique ou d'un dérivé de celui-ci par transformation d'une alcyne terminale avec du dioxyde de carbone, **caractérisé en ce que** la transformation est réalisée en présence d'une base et d'un complexe du cuivre présentant au moins un ligand multidentate, qui présente au moins deux atomes ou groupes d'atomes aptes à une coordination simultanée avec le cuivre, qui sont choisis parmi l'azote, l'oxygène, le soufre, le phosphore et le carbone de type carbène et le complexe du cuivre présentant au moins un autre ligand qui est choisi parmi les phosphines.

2. Procédé selon la revendication 1, un composé de formule
Rₓ-C≡C-H
étant utilisé comme alcyne terminale, dans laquelle
Rₓ représente hydrogène, COOR^{x1}, alkyle, alcényle, cycloalkyle, hétérocycloalkyle, aryle, hétéroaryle ou (R^{x2})₃Si ; et alkyle et alcényle étant non substitués ou présentant un ou plusieurs substituants, par exemple 1, 2, 3, 4 ou 5 substituants R^{x3} ; et cycloalkyle, hétérocycloalkyle, aryle et hétéroaryle étant non substitués ou présentant un ou plusieurs substituants, par exemple 1, 2, 3, 4 ou 5 substituants R^{x4},
R^{x1} étant choisi parmi hydrogène, alkyle, cycloalkyle, hétérocycloalkyle, aryle et hétéroaryle, les quatre derniers radicaux mentionnés étant non substitués ou présentant un ou plusieurs, par exemple 1, 2 ou 3 radicaux, qui sont choisis parmi hydroxy (= OH), mercapto (= SH), NE¹E², C(O)NE¹E², halogène, nitro (= NO₂), nitroso (= NO), formyle (= C(=O)H), alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle et cycloalkyle,
R^{x2} étant choisi parmi alkyle, cycloalkyle, hétérocycloalkyle, aryle et hétéroaryle, les quatre derniers radicaux mentionnés étant non substitués ou présentant un ou plusieurs, par exemple 1, 2 ou 3 radicaux, qui sont choisis parmi hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle et cycloalkyle,
R^{x3} étant choisi parmi halogène, cyano, hydroxy, mercapto, alcoxy, COOH, SO₃H, NE¹E², C(O)NE¹E², acyle, alcoxycarbonyle, cycloalkyle, hétérocycloalkyle, aryle, hétéroaryle, cycloalcoxy, hétérocycloalcoxy, aryloxy, hétéroaryloxy, cycloalcoxycarbonyle, hétérocycloalcoxycarbonyle, aryloxycarbonyle et hétéroaryloxycarbonyle, les groupes cycliques dans les douze derniers radicaux mentionnés étant non substitués ou présentant un ou plusieurs, par exemple 1, 2 ou 3 radicaux qui sont choisis parmi hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle et cycloalkyle,
R^{x4} étant choisi parmi halogène, cyano, nitro, hydroxy, mercapto, alcoxy, COOH, SO₃H, NE¹E², C(O)NE¹E², alkyle, halogénoalkyle, acyle, alcoxycarbonyle, cycloalkyle, hétérocycloalkyle, aryle, hétéroaryle, cycloalcoxy, hétérocycloalcoxy, aryloxy, hétéroaryloxy, cycloalcoxycarbonyle, hétérocycloalcoxycarbonyle, aryloxycarbonyle et hétéroaryloxycarbonyle, les groupes cycliques dans les douze derniers radicaux mentionnés étant non substitués ou présentant un ou plusieurs, par exemple 1, 2 ou 3 radicaux qui sont choisis parmi hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle et cycloalkyle,
E¹ et E² signifiant des radicaux identiques ou différents, choisis parmi hydrogène, alkyle, cycloalkyle ou aryle ; ou E¹ et E² formant, ensemble avec l'atome d'azote auquel ils sont liés, un hétérocycle azoté saturé, qui est non substitué ou qui présente un ou plusieurs groupes alkyle comme substituants.

3. Procédé selon la revendication 1, l'acétylène étant utilisé comme alcyne terminale et de l'acide propiolique et/ou de l'acide acétylènedicarboxylique étant obtenu(s).

4. Procédé selon l'une quelconque des revendications précédentes, le ligand étant un ligand azoté multidentate, qui présente au moins deux atomes d'azote aptes à la coordination avec le cuivre.

5. Procédé selon la revendication 4, le ligand azoté multidentate présentant une structure de formule I 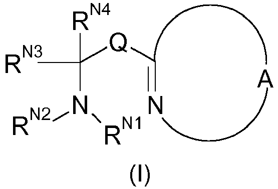 dans laquelle
A ensemble avec le fragment C=N auquel il est lié, forme un hétérocycle de 5 à 7 chaînons, qui peut être annelé avec un, deux ou trois autres cycles ;
Q représente une liaison chimique ou un groupe formant un pont comprenant un, deux ou trois atomes ; la liaison chimique ou le groupe formant un pont pouvant être un constituant partiel ou complet d'un ou de plusieurs cycles ; dans le cas d'un groupe cyclique Q, celui-ci pouvant être annelé avec le cycle A ;
R^{N1} représente hydrogène ou
R^{N1} forme une liaison chimique avec R^{N4},
R^{N2} représente alkyle, cycloalkyle ou aryle,
R^{N3} représente hydrogène, alkyle, cycloalkyle ou aryle, alkyle étant non substitué ou portant un radical du groupe formé par cycloalkyle ou aryle ;
R^{N2} et R^{N3} ensemble avec les atomes, auxquels ils sont liés, forment un hétérocycle de 5 à 7 chaînons, qui peut être annelé avec un, deux ou trois autres cycles ;
R^{N4} représente hydrogène ou est absent ou forme une liaison chimique avec R^{N1}.

6. Procédé selon la revendication 4, le ligand azoté multidentate présentant la formule IV 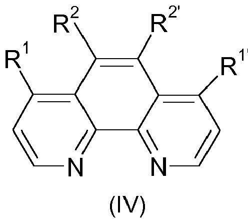
dans laquelle R¹ et R^{1'} représentent, indépendamment l'un de l'autre, hydrogène, hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle, cycloalkyle, hétérocycloalkyle, aryle, hétéroaryle, cycloalcoxy, hétérocycloalcoxy, aryloxy, hétéroaryloxy, arylthio, hétéroarylthio, cycloalcoxycarbonyle, hétérocycloalcoxycarbonyle, aryloxycarbonyle ou hétéroaryloxycarbonyle,
les groupes cycliques dans les quatorze derniers radicaux mentionnés étant non substitués ou présentant un ou plusieurs radicaux qui sont choisis parmi hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle, cycloalkyle, hétérocycloalkyle, aryle, hétéroaryle, cycloalcoxy, hétérocycloalcoxy, aryloxy, hétéroaryloxy, cycloalcoxycarbonyle, hétérocycloalcoxycarbonyle, aryloxycarbonyle, hétéroaryloxycarbonyle,
E¹ et E² signifiant des radicaux identiques ou différents, choisis parmi hydrogène, alkyle, cycloalkyle ou aryle ; ou E¹ et E² formant, ensemble avec l'atome d'azote auquel ils sont liés, un hétérocycle azoté saturé, qui est non substitué ou qui présente un ou plusieurs groupes alkyle comme substituants et
R² et R^{2'} présentent, indépendamment l'un de l'autre, une des significations indiquées pour R¹ et R^{1'}.

7. Procédé selon la revendication 6, les deux radicaux R¹ et R^{1'} dans la formule IV représentant phényle, qui porte le cas échéant 1 ou 2 radicaux, qui sont choisis parmi halogène, alkyle, halogénoalkyle, alcoxy et halogénoalcoxy.

8. Procédé selon la revendication 7, le ligand azoté étant la 4,7-diphényl-1,10-phénanthroline.

9. Procédé selon la revendication 1, ledit au moins un autre ligand étant choisi parmi les triarylphosphines.

10. Procédé selon la revendication 9, ledit au moins un autre ligand étant la tri(p-fluorophényl)phosphine.

11. Procédé selon l'une quelconque des revendications précédentes, le complexe de cuivre étant utilisé comme complexe de cuivre préformé.

12. Procédé selon l'une quelconque des revendications précédentes, une quantité de catalyseur de 0,001% en mole à 20% en mole, calculée sous forme de Cu et par rapport à l'alcyne, étant utilisée.

13. Procédé selon l'une quelconque des revendications précédentes, la transformation étant réalisée en présence d'un solvant, choisi parmi les hydrocarbures aliphatiques, les hydrocarbures aromatiques, les amides, les urées, les nitriles, les sulfoxydes, les sulfones, les alcools, les esters, les carbonates, les éthers et leurs mélanges.

14. Procédé selon l'une quelconque des revendications précédentes, l'acide correspondant à la base présentant une valeur pKₐ à 25°C dans l'eau qui est inférieure d'au moins 3 unités pK à la valeur pKₐ de l'alcyne utilisée.

15. Procédé selon l'une quelconque des revendications précédentes, la base étant choisie parmi les hydroxydes de métal alcalin, les hydroxydes de métal alcalino-terreux, les carbonates de métal alcalin, les carbonates de métal alcalino-terreux, les bicarbonates de métal alcalin, les bicarbonates de métal alcalino-terreux, les oxydes de métal alcalin, les oxydes de métal alcalino-terreux, les phosphates de métal alcalin, les phosphates de métal alcalino-terreux, les hydrogénophosphates de métal alcalin, les hydrogénophosphates de métal alcalino-terreux, les fluorures de métal alcalin, les fluorures de métal alcalino-terreux, les carboxylates de métal alcalin, les carboxylates de métal alcalino-terreux et leurs mélanges.

16. Utilisation de complexe de cuivre (I), comprenant un ligand azoté multidentate et au moins un ligand de type phosphine, le ligand azoté multidentate présentant la formule IV' 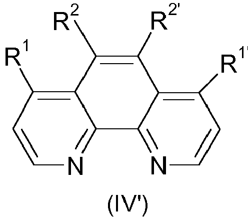
dans laquelle R¹ et R^{1'} représentent, indépendamment l'un de l'autre, hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle, cycloalkyle, hétérocycloalkyle, aryle, hétéroaryle, cycloalcoxy, hétérocycloalcoxy, aryloxy, hétéroaryloxy, arylthio, hétéroarylthio, cycloalcoxycarbonyle, hétérocycloalcoxycarbonyle, aryloxycarbonyle ou hétéroaryloxycarbonyle,
les groupes cycliques dans les quatorze derniers radicaux mentionnés étant non substitués ou présentant un ou plusieurs radicaux qui sont choisis parmi hydroxy, mercapto, NE¹E², C(O)NE¹E², halogène, nitro, nitroso, formyle, alkyle, halogénoalkyle, alcoxy, halogénoalcoxy, alkylthio, halogénoalkylthio, alkylcarbonyle, alkylcarbonyloxy, alkylcarbonylthio, halogénoalkylcarbonyle, alcoxycarbonyle, cycloalkyle, hétérocycloalkyle, aryle, hétéroaryle, cycloalcoxy, hétérocycloalcoxy, aryloxy, hétéroaryloxy, cycloalcoxycarbonyle, hétérocycloalcoxycarbonyle, aryloxycarbonyle, hétéroaryloxycarbonyle,
E¹ et E² signifiant des radicaux identiques ou différents, choisis parmi hydrogène, alkyle, cycloalkyle ou aryle ; ou E¹ et E² formant, ensemble avec l'atome d'azote auquel ils sont liés, un hétérocycle azoté saturé, qui est non substitué ou qui présente un ou plusieurs groupes alkyle comme substituants et
R² et R^{2'} présentent, indépendamment l'un de l'autre une des significations indiquées pour R¹ et R^{1'} ou hydrogène, comme catalyseur de carboxylation pour des alcynes terminales.

17. Utilisation de complexes de cuivre (I) selon la revendication 16 de formule générale
(N,N)CuLₙ⁺X⁻
dans laquelle
(N,N) représente un N,N-ligand bidentate de formule IV',
L représente un ligand de type phosphine,
n vaut un nombre entier de 1 à 3, et
X⁻ représente l'équivalent d'un anion,
comme catalyseur de carboxylation pour des alcynes terminales.
